# EUROPEAN PATENT APPLICATION

(11) **EP 4 684 879 A2**
(43) Date of publication of application: **28.01.2026**
(21) Application number: 25222651.9
(22) Date of filing: 23.10.2020
(51) Int. Cl.: B01L 3/00

(54) **NANOPORE SENSING DEVICE**

(30) Priority: 25.10.2019 GB 201915480
(62) Divisional of application: 20807669.5
(71) Applicant: Oxford Nanopore Technologies PLC, Oxford OX4 4DQ (GB)
(72) Inventor: ORTIZ BAHAMON, Pedro Miguel, Oxford, OX4 4DQ (GB); MACKETT, Paul, Raymond, Oxford, OX4 4DQ (GB); GREASTY, Robert, Oxford, OX4 4DQ (GB); MCKENDRY, Jonathon, Edward, Oxford, OX4 4DQ (GB); HYDE, Jason, Robert, Oxford, OX4 4DQ (GB); JACKSON, Mark, David, Oxford, OX4 4DQ (GB); WILTSHIRE, Richard, Kenneth, John, Oxford, OX4 4DQ (GB); DAVIES, Rhodri, Rhys, Oxford, OX4 4DQ (GB); HYLAND, Mark, Oxford, OX4 4DQ (GB); CLARKE, James, Anthony, Oxford, OX4 4DQ (GB); SANGHERA, Gurdial, Singh, Oxford, OX4 4DQ (GB)
(74) Representative: EIP

(57) **Abstract**

The invention relates to a method of forming a sensing device for supporting a plurality of nanopores upon an array of wells. The method involves providing a substrate, said substrate having a surface having an array of electrodes located thereon for connecting to or for configuring upon an electronic circuit. Separately, a well array structure is provided, which has an array of walls defining through-holes for defining wells. The substrate and well array structures are aligning said array of electrodes define, at least in part, a portion of the bases of respective wells at the bottom of the through holes. The resulting sensing device overcomes problems with known sensing devices by employing a substrate and/or well array structure, or hybrid thereof, that employs alternative materials or manufacturing processes.

## Description

### Background

The invention relates, in general, to a device for nanopore sensing, having an array of nanopore structures configurable as nanopore sensors, as well as methods for fabricating a device for nanopore sensing, having an array of nanopore structures.

Nanopore sensors have been developed for sensing a wide range of species, including single molecules such as polymer molecules. A known nanopore sensor device is a MinION^{™}, manufactured and sold by Oxford Nanopore Technologies Ltd. The nanopore-based sensing therein employs the measurement of ionic current flow through a biological nanopore located in a highly resistive amphiphilic membrane. The MinlION^{™} has an array of nanopore sensors. As a molecule, such as a polymer analyte e.g. DNA, is caused to translocate a nanopore, measurement of the fluctuations in ionic current may be used to determine the sequence of the DNA strand. Nanopore devices for detection of analytes other than polynucleotides such as proteins are also known from WO2013/123379, hereby incorporated by reference in its entirety.

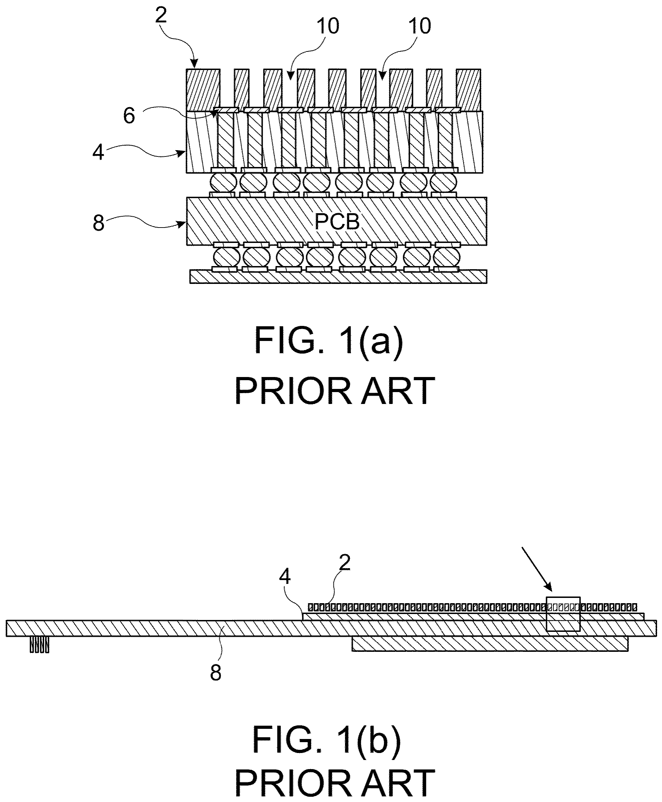

A schematic of a component of known nanopore device, such as the MinION^{™}, is shown in Figures 1(a) and 1(b). Figure 1(a) is an close-up sectional view having a support 2, which is formed by laminating and patterning a dry film resist, which is a photoresist, onto a silicon wafer 4, represented by a through-silicon via (TSV) layer containing an array of electrodes 6 used for electrochemical testing. When the dry film resist is patterned and then developed out on the wafer it leaves a residual resist on the electrodes 6 on the wafer 4 which requires cleaning. This is followed by a baking step at high temperature to fully cross link the photoresist. The cleaning step is known to be sensitive to contaminants in the chamber and there can be inconsistent surface chemistry between batches during manufacture unless great care is taken to clean and condition the RIE chamber consistently. Once the silicon wafer 4 has been laminated, the whole wafer including the microfluidic structure of the support 2 goes through a bump and dice process. The wafer and microfluidic surface of the support 2 are subjected to multiple bakes, flux cleans, washes, and tape application/removal before the sensor device is ready for mounting on a PCB 8 to which an ASIC and other electronic circuits can be connected. Figure 1(b) shows the component in indicating with an arrow the position of the close-up sectional view of Figure 1(a).

Each bump and dice step affects the surface energy of the microfluidic structure resulting in non-optimal and uncontrolled surface properties. Mounting onto a PCB 8 involves further handling of the microfluidic surface with a vacuum chuck followed by baking and underfill steps. All of the PCB mount steps are expected to affect the surface energy of the microfluidic resist structure leading to sub-optimal device performance. In order to regain the optimal performance of both resist and electrode array, multiple cleaning/conditioning steps are necessary before the device is within specification.

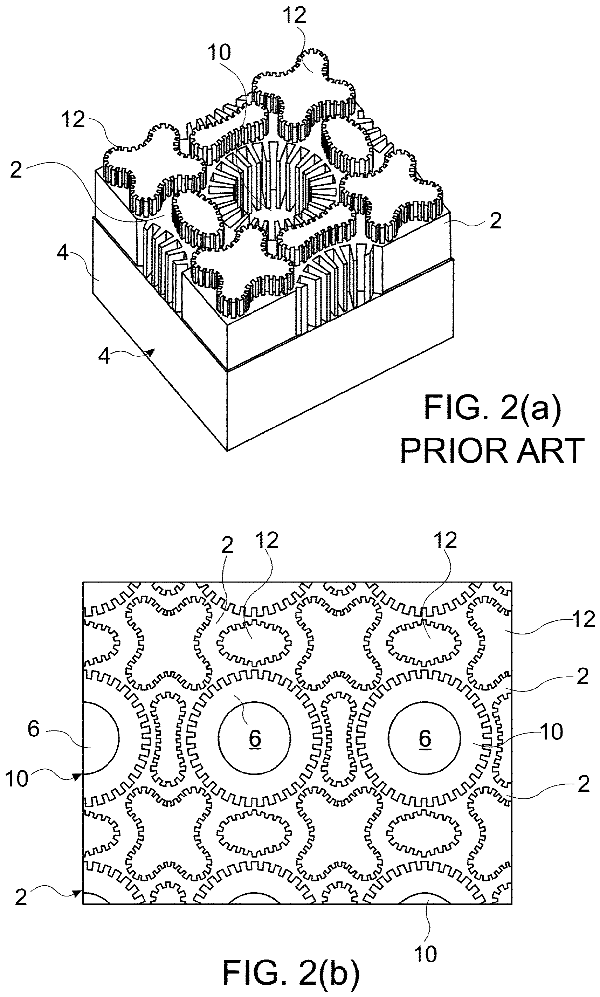

A perspective view of a single well portion of the support 2 is shown in Figure 2(a), with a plan view shown in Figure 2(b) - these drawings show a compartment 10 on one level or layer, which is part of an array of compartments having pillars 12 extending perpendicularly from the surfaces adjacent the compartments. The pillars, therefore, can be said to extend at another level or layer in the support 2. The compartments and pillars have patterns 14 on their surfaces. These Figures are examples taken from WO2014/064443, herein incorporated by reference in its entirety, which describes the requirements and characteristics of such structures e.g. the way in which an amphiphilic membrane is formed and supported by the support 2.

Overall, both the support 2 and electrodes 6 require cleaning before use. This is a timeconsuming process with significant overheads and further complicated by the fact that the two surfaces (sensor layer and electrode) require different conditioning before they are ready for use. Compromises must be made as typical cleaning steps in each case are not cross-compatible. The optimised end state for the surface sensitive materials require different conditioning processes. Electrodes, e.g. platinum - require plasma, acid or base clean, annealing. The support 2 requires a hard bake or plasma clean. Conditioning processes are not compatible across material sets once combined.

### Summary of invention

Overall aspects of the invention seek to reduce the complexity of the manufacture of sensor layers like those disclosed in WO2014/064443, although it is not limited to such methods and structures. Moreover, the invention seeks to provide alternative methods and structures that provide comparable microfluidic functionality while mitigating the problems associated with known manufacturing processes, such as the detriment to the surface energy of the microfluidic structure and/or electrodes. Aspects of the present invention can also serve to reduce material costs and manufacturing costs. Manufacturing techniques and improved structures serve to maintain the quality and performance of the final product.

It is an object of the invention to provide alternative approaches to fabricating nanopore sensor arrays having a plurality of nanopore sensors and components thereof.

When viewed from one aspect the invention provides a method of forming a sensing device for supporting a plurality of nanopores upon an array of wells, the method comprising:
providing a substrate, said substrate having a surface having an array of electrodes located thereon for connecting to or for configuring upon an electronic circuit,
separately providing a well array structure, having an array of walls defining through-holes for defining wells, and
aligning said through-holes and said array of electrodes such that said well array structure becomes part of the substrate and such that said electrodes define, at least in part, a portion of the bases of respective wells.

In the step of aligning said through-holes and said array of electrodes, the well array structure can be bonded to the substrate. In the process of bonding the well array to the substrate the surface of the substrate can be provided with a preparation layer to which the well array can be bonded. The preparation that can include an adhesive, or can be an intermediate material that is processed to provide a mating surface for the well array. The mating surface, for example, can be smoothed or flattened to optimise the connection between the substrate and the well array. In the process of preparing the substrate the electrodes can be covered, at least in part, by the preparation layer. However, after the well array is attached and secured in place, a cleaning process can be used to remove any material of the preparation layer covering the electrodes. The cleaning process can include at least one of plasma cleaning, chemical etching or deep reactive ion etching.

Thus, it will be seen by those skilled in the art that in accordance with at least some examples of the invention a nanopore sensing device may be formed by providing a substrate with electrodes and separately providing a well array structure. This avoids components from being exposed or subjected to known fabrication techniques that include (i) chemical treatment and/or (ii) heating to a temperature which gives rise to outgassing from ancillary components of the sensor, the results of which can compromise the surface energy of the wells and/or contaminate electrodes so affect their performance.

When the through-holes are aligned with the electrodes, each electrode can define at least part of the base of a well. The electrode can form all of the base of the corresponding well, or the base could also comprise part of the substrate adjacent the electrode. Each electrode can be associated with a single well. A well can have only one electrode.

The electrodes on the substrate may be arranged to be attached to a further electronic circuit - e.g. by provision of suitable contact pads or other electrical connectors - for detecting the aforementioned electrical signals. Additionally, or alternatively, an electronic circuit may be formed on the substrate. In some examples, the method can comprise providing an electronic circuit formed independently of said well array structure which contacts or comprises said electrodes. This allows the harsh processing involved in forming the electronic circuit to be carried out away from the well array structure. In some examples the electronic circuit is provided on said substrate and said method comprises forming said well array structure onto said substrate. There are a number of different ways in which the well array structure might be formed onto the substrate. In some examples, however, the method comprises moulding the well array structure onto the substrate. Moulding the well array structure onto a substrate which is already provided with an electronic circuit means that problems outlined hereinabove may be avoided.

In some examples the method comprises forming said through-holes in said substrate and forming said electrodes by providing a conductive material in each through-hole to form at least part of a base of a respective well. In accordance with such examples it will be appreciated that it is not essential to provide a pre-formed substrate with electrodes and a pre-formed well array structure and subsequently attaching them to one another; the conductive material may form the electrode which defines (at least partly) the base of the well.

Furthermore whilst the substrate and electrodes may include the electronic circuit, in a subset of such examples said electronic circuit is provided on a further substrate and the method comprises attaching said further substrate to said sensing device such that said electronic circuit is electrically connected to said electrodes.

In some examples providing said well array structure comprises attaching a pre-formed well array structure to said substrate wherein the electronic circuit is provided on a printed circuit board.

The Applicant has recognised a potential significant benefit in providing an electronic circuit on a substrate or further substrate which is attached to a well array structure - that this may enable or facilitate subsequent separation of these components and so recycling of the sensing device and/or re-use of parts thereof. Apart from the obvious environmental benefits associated with this, it may help to reduce costs and so make the technology more accessible.

In accordance with examples of any aspect of the invention the well array structure has a thickness of less than 0.5mm. Similarly, in some examples the wells in the well array structure have a pitch of between about 10 and about 1000 microns, and preferably between about 200 and about 800 microns, and more preferably between about 150 and about 250 microns.
. In examples the through-holes have a diameter of less than 200 microns. The through-holes can have an aspect ratio between the height and the width of between about 2:1 to 50:1.

As mentioned above in accordance with certain examples of the invention the well array structure may be formed by moulding. Such an approach in the specialist context of the nanopore sensing devices set out herein is a radical departure from previous approaches and is considered to be novel and inventive in its own right.

Thus, when viewed from another aspect the invention provides a method of forming a sensing device for supporting a plurality of nanopores upon an array of wells, the method comprising:
providing a substrate having an array of electrodes located on a surface thereof for connecting to or forming part of an electronic circuit,
providing a mould defining an inverse well array structure comprising an array of protrusions on an underside thereof;
depositing a polymer onto the underside of the mould;
aligning the underside of the mould that is filled with polymer against the substrate such that the protrusions are each aligned with a corresponding electrode;
applying a mutual pressure to the substrate and the protrusions to exclude the polymer from at least a portion of the electrodes;
processing the polymer to cure and secure the polymer to the substrate; and
separating the mould from the polymer, whereby an array of wells of the well array structure are formed by through-holes in the polymer corresponding to the protrusions and a single electrode forming at least part of a base of each well.

Thus, it will be seen that in accordance with this aspect of the invention, a well array structure is formed by through-holes in a moulded polymer. Pressure applied to protrusions in the mould allows polymer to be excluded from the electrodes prior to curing which enables the electrodes to be exposed to analyte in use as is necessary for them to function properly.

When viewed from another aspect the invention provides a method of forming a sensing device for supporting a plurality of nanopores upon an array of wells, the method comprising:
providing a substrate having an array of electrodes located on a surface thereof for connecting to or forming part of an electronic circuit,
providing a mould defining an inverse well array structure comprising an array of protrusions on an underside thereof;
aligning the underside of the mould against the substrate such that the protrusions are each aligned with a corresponding electrode;
injecting a polymer between the mould and substrate;
processing the polymer to cure and secure the polymer to the substrate; and
separating the mould from the polymer, whereby an array of wells of the well array structure are formed by through-holes in the cured polymer corresponding to the protrusions and a single electrode forming at least part of a base of each well.

The mould can be made of an elastomer, such as PDMS. The polymer, which can be referred to as a mouldant, can be a non-crosslinked polymer.

It will thus be appreciated that the well array structure could instead be formed by injection of the polymer into the mould which may obviate the need for mutual pressure to exclude the polymer from the electrodes which is used for the cast moulding previously set out. However, in some examples such mutual pressure is applied, during and/or after injection of the polymer, to ensure exclusion of the polymer from the electrodes.

The polymer is processed to cure it and secure it to the substrate. Any suitable process for doing this may be used, dependent on the type of polymer - e.g. exposure to electromagnetic radiation such as ultra-violet light. In some examples such processing comprises heating the polymer.

As explained above, a sensing apparatus - particularly one suitable for supporting a plurality of nanopores - in which the well array structure is moulded from a polymer, e.g. poly(methyl methacrylate), is a significant departure from the known fabrication approach. When viewed from another aspect the invention provides a sensor apparatus comprising:
an electronic circuit;
a substrate;
an array of electrodes located on a surface of the substrate;
a well array structure, moulded from a polymer, comprising a plurality of walls defining through-holes which together with the electrodes define a plurality of wells, such that each electrode forms at least in part, a portion of a single well; and
wherein the array of electrodes form part of the electronic circuit or are connected to the electronic circuit.

The moulded well array structure can be formed independently of the substrate to which it is attached. This can involve forming the mould for a mouldant, such as a polymer, and then attaching it to a substrate, filling the mould with a mouldant before aligning and connecting it to a substrate or aligning it with a substrate and injecting mouldant between the mould and the substrate.

In some examples the protrusions each define in respective through-holes a pattern on the inwardly facing walls thereof which may be used to give specific surface energy characteristics as is explained in more detail in WO2014/064443 with particular reference to Figs. 19 to 25 and associated description. This could therefore for example comprise a periodic undulating structure such as an array of indentations which define fins. The features of such a pattern - e.g. the fins - may be aligned vertically. They may extend from the top to the bottom of the wells but in some examples, they are terminated by a continuous wall portion adjacent said substrate formed by a distal portion of said protrusion having a continuous perimeter.

Thus, in a specific examples the protrusions each define in respective through-holes an array of fins terminated by a continuous wall portion adjacent said substrate formed by a distal portion of said protrusion having a continuous perimeter and when viewed from another aspect the invention provides a sensor apparatus comprising:
an electronic circuit;
a substrate;
an array of electrodes located on a surface of the substrate;
a well array structure comprising a plurality of walls defining through-holes which together with the electrodes define a plurality of wells, such that each electrode forms at least in part, a portion of a single well, wherein said wells each comprise an array of fins terminated by a continuous wall portion adjacent said substrate; and
wherein the array of electrodes form part of the electronic circuit or are connected to the electronic circuit.

The wall portion having a continuous perimeter has been found to improve the exclusion of polymer from the bottoms of the wells rather than becoming trapped between the distal edges of the fins or other pattern features. The continuous perimeter can at least one of: function to maintain the structural integrity of the walls of a compartment; support the fins and their high aspect ratio structure; and inhibit fins from sticking to one another. It facilitates the release of the structure made of cured polymer form the elastomeric mould.

The continuous perimeter can be substantially continuous and have one of more recesses, such as notches, arranged to inhibit liquid pooling across the surface of the electrode.

The moulding methods set out herein could employ a mould supplied from elsewhere. However, in some examples providing the mould comprises depositing a moulding material onto a master well array structure comprising a plurality of wells to form the mould; and separating the mould from the master well array structure. As may be appreciated, producing moulds from a master allows greater quality control - e.g. by facilitating an approach whereby moulds are reused a small number of times before being discarded or recycled to avoid any appreciable degradation thereof and new moulds can then be produced easily. The master could be made from any suitable durable material - e.g. metal. In some examples the master well array structure is fabricated from a silicon wafer. The moulding material, from which the temporary mould is made, may be any suitable material but can be a polymer. In one specific example the moulding material is polydimethylsiloxane.

The mould can be provided with features, such as extensions, on the protrusion. These features can increase and/or manage the movement of mouldant, such as polymer, when the well array structure is being moulded. When improving upon known structures using a moulding technique to form the well array structure the inventors discovered new problems, such as the accumulation of oil at the bottom of a well when the fins no longer extend to the aperture adjacent the electrode, or that the protrusion of the mould that forms the through-hole down to the electrode fails to adequately displace the polymer from the surface of the electrode. Extensions can be provided to form recesses to capture oil in the fin base. Extensions can be formed on the end of the protrusion to manage the flow of polymer at the electrode and keep clear areas to function. An extension can have a pattern. The extension can have a gradient from the edge towards the centre of the protrusion. The gradient can be a tapered triangle shape. The gradient can be a change in height towards the centre. The pattern can have segments, such as wedge-shaped segments. In accordance with any aspect involving moulding the well array structure the polymer used may be any suitable material which can be moulded with sufficiently fine features. In one specific example the polymer is poly(methyl methacrylate).

In some examples of the moulding aspects of the invention wherein the polymer is dissolved in a solvent before being deposited onto the underside of the mould. This may facilitate its release from the mould without compromising its surface energy characteristics. In some examples the polymer can be heated to evaporate the solvent.

In some examples, before positioning the underside of the mould against the substrate, a polymer is deposited onto the substrate. This can be the same polymer as that used in the mould but that is not essential. This may help adhesion between the substrate and the structure moulded thereon.

In some examples the cured polymer is subsequently peelable from the substrate. This facilitates the recycling/re-use referred to above.

In some examples of any aspect of the invention, the method further comprises subsequently cleaning the electrodes. This may ensure that a sufficiently high-quality electrical contact surface is achieved. Such cleaning could be achieved by chemical cleaning and/or using plasma treatment, both known *per se* in the art.

In another example, the method can include forming a sensing device for supporting a plurality of nanopores upon an array of wells, the method comprising: providing a substrate having an array of electrodes located on a surface thereof for connecting to or forming part of an electronic circuit, providing a mould defining an inverse well array structure comprising an array of protrusions on an underside thereof; aligning the underside of the mould against the substrate such that the protrusions are each aligned with a corresponding electrode; injecting a polymer between the mould and substrate; processing the polymer to cure and secure the polymer to the substrate; and separating the mould from the polymer, whereby an array of wells of the well array structure are formed by through-holes in the polymer corresponding to the protrusions and a single electrode forming at least part of a base of each well.

The invention extends to a mould adapted for use in the methods set out above - e.g. one which is produced by a master well array structure as set out above. The invention also extends to a master well array structure as set out above *per se.*

When viewed from another aspect the invention provides a method of forming a sensing device for supporting a plurality of nanopores upon an array of wells, the method comprising: providing a well array structure having an array of walls defining through-holes for forming wells providing a substrate having an array of electrodes located on a surface thereof for connecting to or forming part of an electronic circuit, providing a patterned adhesive surface on at least one of the well array structure or the substrate, wherein said patterned adhesive surface defines a plurality of portions without adhesive; thereafter, aligning the well array structure and substrate such that the electrodes are aligned with the plurality of portions without adhesive and with the through-holes in the well array structure such that the electrodes define, at least in part, a portion of a single well; using the patterned adhesive surface to adhere the well array structure to the substrate. The portions without adhesive can be areas or voids in the patterned adhesive surface that expose the electrodes to the through-holes in the well array structure. The plurality of portions without adhesive can be described as having holes that align with the apertures at the end of the through-holes in the well array structure.

Thus it will be seen by those skilled in the art that in accordance with this aspect of the invention a patterned adhesive is used to adhere a well array structure and a substrate carrying electrodes aligned with through-holes to form wells of a nanopore sensing device of the type described herein. This may provide similar benefits to those set out above in respect of other aspects of the invention in the electrodes may be fabricated separately from the well array structure to avoid exposing the latter to the processing required for the former and so potentially compromising the characteristics of the device produced. It also facilitates subsequent separation of these components for reuse/recycling as previously explained.

In some examples an electronic circuit is provided which is formed independently of said well array structure and which contacts or comprises said electrodes. As previously discussed, the electrodes could form an integral part of the electronic circuit which measures the electrical signals from them. This may be advantageous in obviating the need to connect the electrodes to an electrical circuit using costly through-silicon vias (TSVs) which has been adopted as the standard approach in fabricating nanopore sensing devices. Alternatively, such a circuit could be on a separate substrate, further facilitating its fabrication using processes that would compromise the well array structure and/or production by another entity.

The well array structure may be adhered to the substrate in any suitable way depending on the adhesive used. For example, the adhesive surface may be heated and/or exposed to electromagnetic radiation. Where it is heated, it can be heated only to a temperature below the outgassing temperature of components of the electronic circuit, such as polymers. This realises the advantage previously set out of such fabrication approaches.

In some examples providing the patterned adhesive surface comprises exposing a photo-patternable adhesive to light. Advantageously exposing the photo-patternable adhesive to light provides said plurality of portions without adhesive.

In one example the adhesive surface is integrated into one of the well array structure or the substrate. In another example the patterned adhesive surface is applied as an independent layer to the well array structure or the substrate. In such examples the adhesive layer may be provided, and a pattern formed in said adhesive layer, thereby forming said independent patterned adhesive surface layer. Examples thereof comprise transferring the independent layer onto the well array structure or the substrate using an intermediate surface. The intermediate surface may, for example, be formed from polydimethylsiloxane.

Other examples comprise providing the patterned adhesive surface layer on the well array structure or the substrate using an aerosol spray or micro-drop dispenser; or adhering the well array structure to the substrate using capillary action of an adhesive to form said patterned adhesive surface layer.

Additionally or alternatively, the well array structures and/or electrodes located at the base of the wells can be exposed to plasma. Exposing the electrodes to plasma functions to clean the surface of the electrodes. When the surface of the well array structure is exposed to plasma changes the surface properties to be, at least temporarily, adhesive such that additional layers applied to the well array structure will cause them to stick or bond.
When the well array structure and the electrodes are exposed at the same time the use of plasma simultaneously cleans the electrodes and makes the surface of the well array structure sticky, providing it with adhesive properties, ready for accepting a further layer on top. When viewed from another aspect the invention provides a method of forming a sensing device for supporting a plurality of nanopores upon an array of wells, the method comprising: providing a substrate having an electrically conductive layer on a backside thereof; forming vias penetrating through the substrate; at least partially filling said vias with a conductive filling material to form electrodes; removing a portion of the electrically conductive layer to form electrical contacts each in contact with a respective electrode for connecting to an electronic circuit; and removing part of the front side of said substrate to form an array of wells, such that each electrode forms at least in part, a portion of a single well.

The invention extends to corresponding sensor apparatus and so when viewed from another aspect the invention provides a sensor apparatus comprising: a substrate comprising a plurality of vias at least partially filled with conductive filing material thereby forming an array of electrodes; a well array structure formed from the substrate to define an array of walls defining through-holes which together with the electrodes define a plurality of wells, such that each electrode forms at least in part, a portion of a single well; and a plurality of electrical contacts on a backside of the substrate each in contact with a respective electrode for connecting to an electronic circuit.

Thus it will be seen by those skilled in the art that in accordance with these aspects of the invention the electrodes are provided on the substrate by filling vias with a conductive material and the well structure is formed by removing part of the substrate - e.g. by machining it. Filled vias become conductive vias. This may achieve the advantages set out in accordance with other aspects of the invention of avoiding exposure of the well array structure to unfavourable processing. It also obviates the need to use TSVs, bringing further potential benefits. The contacts on the back side of the substrate can be used to connect to an electronic circuit and thus in some examples the method further comprises providing an electronic circuit formed independently of said well array structure which contacts said electrodes. In some examples the apparatus comprises an electronic circuit connected to the electrical contacts. This supports the aforementioned advantages of independent processing and facilitating reuse and recycling.

In some examples wherein the substrate can be a thermoplastic substrate.

In some examples the method comprises applying a coating to said conductive filling material to provide said electrode. This could, for example, be any high quality, electrically conductive coating such as platinum, silver or an alloy thereof to give superior electrical properties compared to those of the conductive filler material itself which might, for example, be a carbon paste.

In accordance with the foregoing aspect, part of the front side of the substrate is removed to form the wells. In some examples removing part of the front side of said substrate comprises ablating the substrate. Examples may use a laser to remove part of the front side of said substrate. Examples may use scanned mask imaging to form the well array structure.

In some examples the method comprises forming the vias in first and second steps wherein the first step comprises forming a first part of the via and the second step comprises forming a second part of the via having a greater cross-sectional area than the first part of the via. In such examples only the first part of the via might be filled with the conductive filling material. This may provide a convenient way to form a structure which gives rise to a well formed from the second, wider part of the via, which has an electrode at the bottom formed by the first, narrower part filled with conductive material.

In some examples forming said well array structure comprises initially forming a pillar layer and subsequently forming a layer of wells between respective pillars.

In some examples partially filling said vias with said conductive filling material comprises forming the conductive filling material into protrusions on the back side of the substrate.

In some examples the substrate and well array structure are formed from a laser machined plastic material. This is considered to be novel and inventive in its own right and thus when viewed from another aspect the invention provides a method of forming a sensing device for supporting a plurality of nanopores upon an array of wells, the method comprising:
providing a substrate formed from a laser machined plastic material, said substrate having a surface having an array of electrodes located thereon for connecting to or for configuring upon an electronic circuit;
separately providing a well array structure formed from a laser machined plastic having an array of walls defining through-holes for defining wells; and
aligning said through-holes and said array of electrodes such that said well array structure becomes part of the substrate, and such that said electrodes define, at least in part, a portion of the bases of respective wells.

With reference to previous discussion, in at least some aspects of the invention nanopore sensing devices may be produced without employing TSVs thereby enabling more cost-effective production. The Applicant has recognised that the techniques set out above for forming electrodes by filling vias with conductive filling material can be used to produce a substrate to which a well array structure may be attached in order to bring the advantages of independent processing and easier disassembly which aspects of the invention may provide.

When viewed from another aspect therefore the invention provides a method of forming a sensing device for supporting a plurality of nanopores upon an array of wells, the method comprising: providing a substrate having an electrically conductive layer on at least a frontside thereof; forming vias penetrating through the substrate; filling said vias with a conductive filling material; removing a portion of the electrically conductive layer to form a plurality of electrodes on the frontside of said substrate attaching to the frontside of the substrate a well array structure comprising an array of walls defining through-holes which together with the electrodes define a plurality of wells, such that each electrode forms at least in part, a portion of a single well.

Vias can be filled with a conductive filling material, to form a conductive via using, for example, a screen-printing technique. When the via is formed a capillary stop can be implanted around the via aperture to inhibit uncontrolled spread of screen-printed material. The capillary stop can be a groove substantially surrounding the via aperture.

In some examples an electronic circuit formed independently of said well array structure is provided on a backside of the substrate wherein said electronic circuit is electrically connected to said electrodes by said vias. This means that a single substrate may carry the electrodes and the electronic circuit such that a complete sensing device for supporting nanopores can be produced simply by adding a well array structure to such a substrate. Such arrangements may allow for a convenient and low-cost fabrication approach whereby the substrate is formed using relatively more straightforward manufacturing techniques without compromising the wells and the more critical well array structure fabrication can be carried out independently.

In another example, a substrate is formed having conductive vias by initially forming vias in a sheet of plastic material and subsequently coating said sheet, including the surface of the via, to implement a conductive via.

In another set of examples, said substrate has an electrically conductive layer on a backside thereof, the method further comprising removing a portion of the backside electrically conductive layer to form electrical contacts each in electrical contact with a respective electrode for connecting to an electronic circuit. The method may this comprise providing an electronic circuit formed independently of said well array structure and connecting said electronic circuit to said electrical contacts on said substrate. This provides flexibility in allowing the wells, the electrodes and the electronic circuit to be produced independently of one another, thereby permitting optimal processing of each and enhancing the opportunity to separate components at the end of their useful life for reuse/recycling. The independently formed electronic circuit could be attached before, after or at the same time as the well array structure is attached. In all such cases the advantages of fabricating the electrodes and the electronic circuit without contaminating the well array structure can be realised.

In some examples attaching the well array structure onto the front side of the substrate comprises: providing a patterned adhesive surface on at least one of the well array structure or the substrate, wherein said patterned adhesive surface defines a plurality of portions without adhesive; thereafter, aligning the well array structure and substrate such that the electrodes are aligned with the plurality of portions without adhesive and with the through-holes in the well array structure such that the electrodes define, at least in part, a portion of a single well; and using the patterned adhesive surface to adhere the well array structure to the substrate.

The portions without adhesive can be areas or voids in the patterned adhesive surface that expose the electrodes to the through-holes in the well array structure.

As will be recognised, in such examples the well array structure is attached to the substrate using one of the methods set out above. The advantages and optional features thereof are therefore applicable to such examples.

In some examples attaching the well array structure onto the front side of the substrate comprises: providing a mould defining an inverse well array structure comprising an array of protrusions on an underside thereof; depositing a polymer onto the underside of the mould; aligning the underside of the mould that is filled with polymer against the substrate such that the protrusions are each aligned with a corresponding electrode; applying a mutual pressure to the substrate and the protrusions to exclude the polymer from at least a portion of the electrodes; processing the polymer to cure and secure the polymer to the substrate; and separating the mould from the polymer, whereby an array of wells of the well array structure are formed by through-holes in the polymer corresponding to the protrusions and a single electrode forming at least part of a base of each well.

As will be recognised, in such examples the well array structure is attached to the substrate using one of the moulding methods set out above. The advantages and optional features thereof are therefore applicable to such examples.

In some examples filling said vias with said conductive filling material comprises forming the conductive filling material into protrusions on the frontside and/or the backside of the substrate. In some examples, the conductive filing material comprises carbon and/or silver paste. In some examples of these aspects the electrically conductive layer comprises platinum and/or silver.

In some examples of any aspect of the invention the method comprises adding an aqueous solution to the wells of the well array structure. The aqueous solution can comprise a polar medium. The method can further comprise forming a plurality of membranes comprising amphiphilic molecules in the wells of the well array structure to encapsulate the aqueous solution therein, thereby forming an array of discrete electrically isolated volumes. The amphiphilic molecules can comprise a hydrophilic head group and a hydrophobic tail group.

In some examples of any aspect of the invention the substrate is a thermoplastic substrate.

In some examples of any aspect of the invention the substrate initially has a uniform thickness.

The invention extends to any sensing device produced by one of the methods set out hereinabove. As will be appreciated by those skilled in the art, the sensing devices disclosed herein have features which make them novel and inventive in their own right and when viewed from another aspect the invention provides a sensor apparatus comprising: an electronic circuit; a substrate; an array of electrodes located on a surface of the substrate; a well array structure comprising a plurality of walls defining through-holes which together with the electrodes define a plurality of wells, such that each electrode forms at least in part, a portion of a single well; and wherein the array of electrodes form part of the electronic circuit.

Thus, it may be appreciated that in accordance with this aspect of the invention, the electrodes at the bases of the wells form part of an electronic circuit. This may bring manufacturing advantages as already detailed and also obviates the need to provide a costly TSV as in existing designs.

When viewed from an aspect the invention provides a sensor apparatus comprising: an electronic circuit; a thermoplastic substrate; an array of electrodes located on a surface of the substrate; a well array structure comprising a plurality of walls defining through-holes which together with the electrodes define a plurality of wells, such that each electrode forms at least in part, a portion of a single well; and wherein the array of electrodes is connected to the electronic circuit.

As with the previous aspect of the invention, such arrangements may be advantageous in obviating the need to provide a costly TSV as in existing designs, since a thermoplastic substrate is provided on which the electrodes are disposed. This represents a departure from the technical prejudice in the art which had taken a silicon substrate to be essential.

It will be appreciated from the foregoing inventive principles and advantages thereof that when viewed from another aspect the invention provides a sensor apparatus comprising:
a substrate comprising a plurality of vias penetrating through the substrate, at least partially filled with conductive material and forming a plurality of electrodes;
a well array structure adhered to a front side of the substrate, said well array structure comprising an array of walls defining through-holes which together with the electrodes define a plurality of wells, such that each electrode forms at least in part, a portion of a single well; and
an electronic circuit on or connected to a backside of the thermoplastic substrate.

In some examples the substrate has connectors for making electrical contact with an electronic circuit. These may comprise protrusions from said vias. The protrusions can be formed, at least in part, from the conductive material.

In some examples the substrate comprises an electronic circuit formed onto its backside.

In some examples of the apparatus in accordance with any aspect of the invention the apparatus further comprises an aqueous solution and a plurality of membranes comprising amphiphilic molecules in the wells of the well array structure to encapsulate the aqueous solution therein, thereby forming an array of discrete electrically isolated volumes. This enables the apparatus to provide the Applicant's established and highly successful nanopore sensing technology. The aqueous solution can be a polar medium. The amphiphilic molecules can comprise a hydrophilic head group and a hydrophobic tail group.

Further in some examples of the apparatus in accordance with any aspect of the invention the well array structure comprises a base and partitions extending from the base that define the wells and constrain an aqueous solution in a well from contacting aqueous solution in neighbouring wells. The partitions can comprise inner portions defining inner recesses and outer portions. The inner recesses can have surfaces having a patterning comprising a plurality of indentations that extend outwardly of the inner recess. The outer portions can have surfaces having a patterning comprising a plurality of indentations that extend outwardly of the inner portions. The outer ends of the partition can extend in a common plane.

Overall, the methods and substrates herein provide for improved microstructures, such as polymer microstructures, with high aspect ratio features, that due to the nature of the materials involved, can be used to pattern sensor devices with minimal fouling of an electrochemical transducer's surface. The cost of the materials and equipment involved can be considerably lower than known techniques (such as SU8 lithography, LIGA, DRIE) traditionally used to fabricate this type of structure. The fabrication methods, which include the independent formation of components to minimising the exposure of critical materials to other processes (thermal or chemical) required for the assembly of the device.

While the examples herein have been described and are suitable as substitute components and methods of manufacture of well array structures for a MinION (RTM) the invention is not limited thereto and can be applied to any device requiring comparable features.

Using the techniques taught herein, a sensing device can be fabricated with microstructures having high aspect ratios that creates a morphology that combine with the intrinsic surface energy of the structural material to form a support having an array of discrete, electrically insulated wells for holding aqueous volumes that serve as base for membrane formation and subsequent nanopore insertion.

When viewed from a further broad aspect the invention provides a method of forming a sensing device for supporting a plurality of nanopores upon an array of wells, the method comprising providing a well array structure, having an array of walls defining through-holes for forming wells, onto a substrate, said substrate having a surface having an array of electrodes located thereon for connecting to or forming part of an electronic circuit, such that said through-holes and said electrodes are aligned and such that each electrode defines, at least in part, a portion of a single well.

### Figures

Known structures have been described above in relation to Figures 1 to 2(b) and now aspects of the invention will now be described, by way of example only, with reference to the remaining drawings in which:
Figure 3 is a schematic cross-sectional representation of a sensing device, or component thereof, having a turreted well array structure connected to a substrate having electrodes at the base of the walls;
Figures 4(a) to 4(f) are cross-sectional views of process steps that can be used to make the component of Figure 3;
Figure 5 is a plan view of a negative mask impression taken from MinION (RTM) product, showing fins having open ends;
Figure 6 is a perspective view a single compartment of an improved well array structure in which the fins are closed at one end, thus shortening the fin length;
Figure 7 is a perspective view a single compartment of an improved well array structure similar to the compartment of Figure 6, in which the fins are shorter;
Figure 8 is a perspective view of a mould having a protrusion with shortened fins that can be used to form the compartment of the well array shown in Figures 6 and 7;
Figure 9 is a perspective view a single compartment of an improved well array structure in which recesses are provided at the base of the compartment;
Figure 10 is a perspective view a single compartment of an improved well array structure in which a pattern is formed on the base of the compartment;
Figure 11 is a perspective view a mould that can for a single compartment of an improved well array structure in which the fins are closed at one end like Figure 6, recesses can be formed like Figure 9 and a pattern can be formed like Figure 10;
Figure 12(a) is a plan view image of a moulded well array structure in which Newton's rings indicate that the film of mouldant remaining is thicker in the centre, while Figure 12(b) is a perspective view of a mould for forming a single compartment of an improved well array structure in which the end of the protrusion has extensions that are configured to improve the exclusion of mouldant;
Figures 13(a) to 13(e) are schematic cross-sectional representations of method steps for forming a sensing device, or component thereof, having a turreted well array structure connected to a substrate that is coated, drilled, filled and etched before the well array structure is connected;
Figure 14 is a cross-sectional view of a conductive via in a substrate;
Figures 15(a) and (b) are cross-sectional views of vias through a substrate prior to coating with a metal film;
Figure 16(a) to (g) are schematic cross-sectional representations of method steps for forming a sensing device from a sheet having a metalised coating on one side, including forming and filling vias and, on the other side, forming a well array structure in the sheet;
Figures 16(g) and (i) are, respectively, a well array structure formed using laser ablation and a close-up view of said well array structure showing the fins and pillars;
Figure 17 is a representation of a screenshot of measurements taken during the insertion of a nanopore into a membrane supported on a sensing structure of the invention;
Figure 18(a) is a cross section view of a conductive via through a sheet having metal film on both sides, while Figure 18(b) is a cross section view of a conductive via through a sheet having metal film on one side and a screen-printed pad on the other;
Figure 19(a) is a schematic plan view of a track and pad on a surface of a substrate aligned with a sectional view of a conductive via connected to the pad, while Figure 19(b) is a schematic view of a cross-section and plan view of a pad having a capillary stop; and
Figure 20 is a table and corresponding properties of sheet materials.

### Description

Overall, the examples of the invention seek to provide alternative structures and/or processes that mitigate one or more problems with known structures and methods used in fabricating sensing devices, such as nanopore sensing devices.

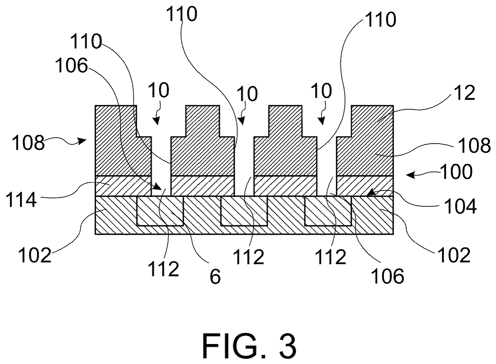

Figure 3 shows a sensing device 100, or portion thereof, for supporting a plurality of nanopores upon an array of wells 10 - only 3 of which are shown. The sensing device has a substrate 102 having a surface 104 upon which an array of electrodes 6 are located. The electrodes 6 are exposed at apertures 106 at the bottom of the wells, as viewed. The electrodes can be connected to, or be configured upon, an electronic circuit. Upon the substrate 102 is a well array structure 108 having an array of walls 110 defining through-holes 112 with the apertures 106 for defining the wells 10 when connected to the substrate 102. The well array structure can be connected directly to the substrate 102, although this connection has been shown with adhesive layer 114. Additionally, or alternatively, the connection can use an adhesive surface or other such bonding materials or methods. When connected to the substrate 102 the through-holes 112 and apertures 106 are aligned with the array of electrodes 6 such that the base of the wells 10 of said well array structure, are defined, at least in part, by the electrodes. The base of the wells can also be defined, at least in part, by the surface 104 of the substrate.

To form the sensing device 100, before supporting a plurality of nanopores upon the array of wells, the substrate 102 is provided. This is to say that no further processing or manufacturing steps are required to the substrate, or well array structure, after it is connected to the well array structure that will negatively impact the performance of the sensing device 100.

The well array structure 108 is also provided separately, having pillars 12 on one side and apertures 106 on the other. By providing the substrate and well array structure separately they can be manufactured without the conditions in which they are manufactured having any detriment upon the other component.

The substrate 102 can have an electronic circuit on the surface 104, or the substrate can be a printed circuit board. The electronic circuit and electrodes can be provided on the surface of an application specific integrated circuit (ASIC).

The separate provision of the well array structure can include forming the well array structure on the surface of the substrate 104 i.e. after the substrate 102 has been provided the surface 104 can be used as a base upon which the well array structure is formed. The well array structure can be moulded and then applied to the substrate or can be moulded directly onto the substrate surface 104.

The electrodes 6 are shown in Figure 3 as part of the surface 104 of the substrate 102. In an alternative example, the substrate can be provided with through-holes and filled with a conductive material to provide a via and the exposed portion of the via functions as an electrode forming, at least in part, a base of a well 10 in the aperture 106 of the well array structure.

The well array structure can have a thickness of between 10 microns and 1000 microns, and preferably between 10 microns and 500 microns, and more preferably between 100 microns and 500 microns. The depth of the wells can be between about 5 microns and about 1500 microns, and preferably between 10 microns and 500 microns, and more preferably between 100 microns and 500 microns. The pitch between the wells in the well array structure can be between about 10 and about 1000 microns, and preferably between 10 microns and 500 microns, and more preferably between 20 microns and 100 microns. Through-holes can have a diameter of less than 200 microns, and preferably between 10 microns 200 microns

After the sensing device 100 is formed the wells can be populated with liquid and membranes formed across the wells before inserting nanopores in the membrane. The membranes can include (i) amphiphilic membranes, and support biological nanopores, (ii) solid-state membranes, and have solid-state nanopores, (iii) solid state membranes and support a biological nanopore in a hole within said solid-state membrane.

The sensing device 100 can be fabricated in a number of ways. The function of the sensing device can also be implemented by incorporating a mixture of the different modular components described herein - such as the substrate 102 and well array structure 108, which can be fabricated themselves in different ways using different materials. Overall, however, the important elements i.e. the substrate 102 and the well array 108 are connected together in the later stages of fabricating the sensor. In many of the examples herein the well array structure 108 can be removably attached to a substrate such that it can be removed and replaced with a new substrate, thus making the sensing device recyclable. In one example, forming the sensing device 100 includes forming the well array structure 108 having the through-holes 112 and further providing a patterned adhesive surface, which can be an adhesive layer 114, on at least one of the well array structure or the substrate, wherein said patterned adhesive surface defines a plurality of portions without adhesive.

The portions without adhesive can be areas such as holes or voids in the patterned adhesive surface that expose the electrodes to the through-holes in the well array structure. The patterned adhesive surface can also be one of the surface of the well array structure 108 or substrate 102 that have been prepare or treated to give the connecting surface adhesive properties, for example by exposing the surface to UV light.

The well array structure is aligned and connected to the substrate using the adhesive layer such that the array of electrodes 6 located on a surface thereof define, at least in part, a portion of a single well.

The substrate 102 supports or incorporates an electronic circuit having electrodes 6 and is formed independently of said well array structure which contacts or comprises said electrodes. The adhesive surface of the adhesive layer 114 can be heated to assist adhering the well array structure to the substrate. The heating can be limited to a temperature below the outgassing temperature of components of the electronic circuit. Electromagnetic radiation can, additionally or alternatively, be used to adhere the well array structure to the substrate 102. The adhesive layer 114 may be a photo-patternable adhesive that is exposed to light prior to adhesion. Additionally or alternatively, the well array structures and/or electrodes located at the base of the wells can be exposed to a plasma treatment that functions to at least one of: clean exposed surface and temporarily change the properties of exposed surfaces to become adhesive in nature e.g. sticky.

As shown in Figure 3, the adhesive layer 114 can be an independent layer to the well array structure or the substrate. It can, however, be integrated into one of the well array structure or the substrate. Or it can be a region of the well array and/or the substrate that has been modified to have adhesive properties, such as by heating.

One example method of the invention allows the well array structure 108 to be fabricated separately from the substrate 102 and then for them to be combined after they have been optimally processed. The method is composed of (i) making a stand-alone microfluidic well arrays structure 108 from thick film photoresist about 120 microns thick, which is available from TOKYO OHKA KOGYO CO., LTD. (TOK), (ii) making a stand-alone patterned adhesive layer 114 with at least one of photo-definable adhesives (also available from TOK), laser cut adhesives or liquid adhesive and controlled wetting of the substrate/resist interface, or preparing the surface of the well array structure or the substrate to have adhesive properties (iii) alignment and placement of the adhesive on to the substrate 102, e.g. by robotic placement, (iv) alignment and placement of the microfluidic structure 108, e.g. by robotic placement, and (v) post-processing to cross link the materials using, for example, UV exposure or heating.

In more detail, the photoresist can take the form of thick film laminates that are laminated onto a sacrificial layer. The sacrificial layer can, for example, be Omnicoat (RTM) or polystyrene, which can be spun onto a Si wafer. The use of the silicon layer is just one technique that enables the well array structure to be fabricated independently of the substrate to which connects. As described herein, making the components separately avoids a manufacturing process of one component adversely influencing the other.

To reduce sticking to the mask during the lithography step either a post-process heating process is adopted or the mask can be treated beforehand to avoid sticking.

To minimise stress in the laminate occurring because of the connection with other surfaces post-processing temperature control is adopted.

When the adhesive layer is made from photo-definable adhesives a plurality of vias are provided, which align to the apertures 106 in the microfluidic structure such that they function to extend the through-holes 112. The adhesive layer 114 can be selected to have the same surface energy as the well array structure 108. By way of example it can be obtained from TOK (NC-00075S; thicknesses of 20um and 50um).

Other methods of forming stand-alone patterned adhesive layers include (but are not limited to): using a laser machine to ablate appropriate vias in pre-existing adhesive materials such as pressure sensitive adhesive layers (PSA), using liquid adhesive and controlled wetting at the interface of well array structure and substrate, using an aerosol spray or micro-drop dispenser, using capillary action with a liquid adhesive. The adhesive material can have the same surface energy as the well array structure.

Ensuring complete cross-linking can be achieved by baking. The temperature of the bake is limited to be below the outgas temperature of the substrate 102, which can be polymers present on a PCB for example.

After independent formation of the well array structure 108 on, for example, a Si wafer it is removed. The removal step can include a toluene lift-off before attachment to the substrate using adhesion.

The adhesive layer can be an adhesive surface i.e. the well array structure has adhesive properties on the surface adjacent the apertures 108. This can be achieved by not hard-baking the well array structure and leaving residual solvents and/or uncross-linked material. Heat and pressure can be used to enable adhesion.

The well array structure can have a thin film of adhesive transferred on to its surface in a stamp-transfer process. The stamp can be made of polydimethylsiloxane (PDMS). The stamp can have fine bristles to control transfer volumes of adhesive. The surface energy of PDMS is suited to controlling the volume of adhesive pinned to the bristle tops because of its low surface energy. The stamp-transfer is preferentially made to the well array structure 108. This is because the substrate can have electrodes thereon and while the layout and density of the bristles can be arranged to inhibit any transfer of adhesive to the electrode surface on the substrate 102 the application of adhesive to the well array structure mitigates this happening.

In another example, forming the sensing device 100 includes forming the well array structure 108 using a moulding technique. This can achieve independently of the manufacture of the substrate 102. The independent formation of the well array structure can occur either on a reference surface before subsequent removal and application to the substrate or, alternatively, the moulding can take place directly upon the substrate.

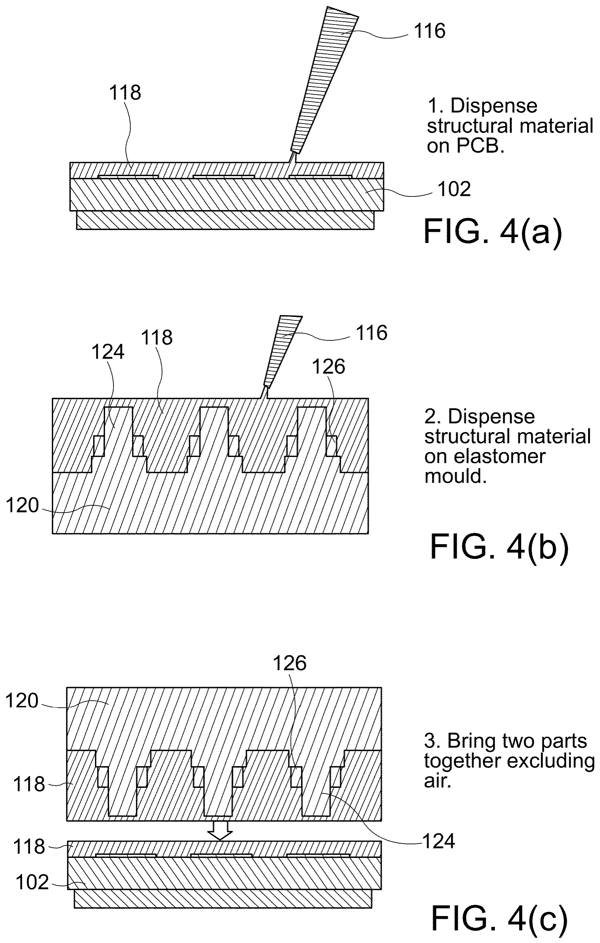

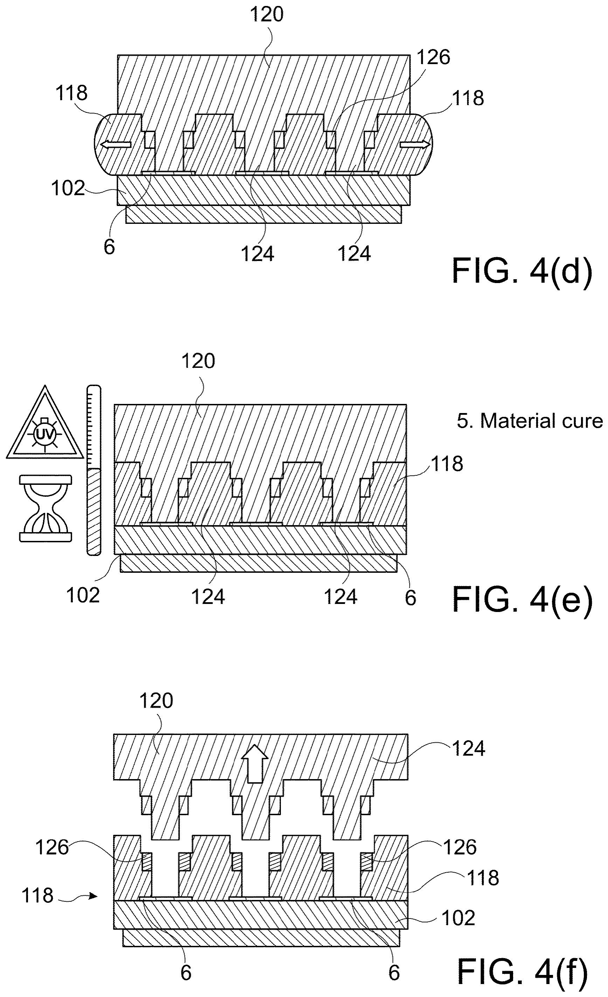

The basic steps of a moulding example are illustrated in Figures 4(a) to 4(f). Initially, a dispenser 116 deposits a mouldant, referred to hereinafter as a polymer 118, by way of example, on the surface of a substrate 102 having electrodes 6. The polymer 118 is distributed evenly over the surface of the substrate.

Separately, in a second step, polymer 118 is dispensed into a mould 120, wherein the mould is a negative of the well array structure 108 to be formed. The mould 120 can be fabricated from a master 122, wherein said master is identical to the well array structure to be formed. In the process of researching and developing the inventors choose to use the well array structure of a current MinION (RTM) product that can be exemplified by the compartments 10 and pillars 12 of the support 2 shown in Figures 2(a) and 2(b), although the examples of the invention are not limited thereto. Alternatively, the mould 120 can be fabricated from scratch without a master. In this way imperfections can be mitigated when the mould is created.

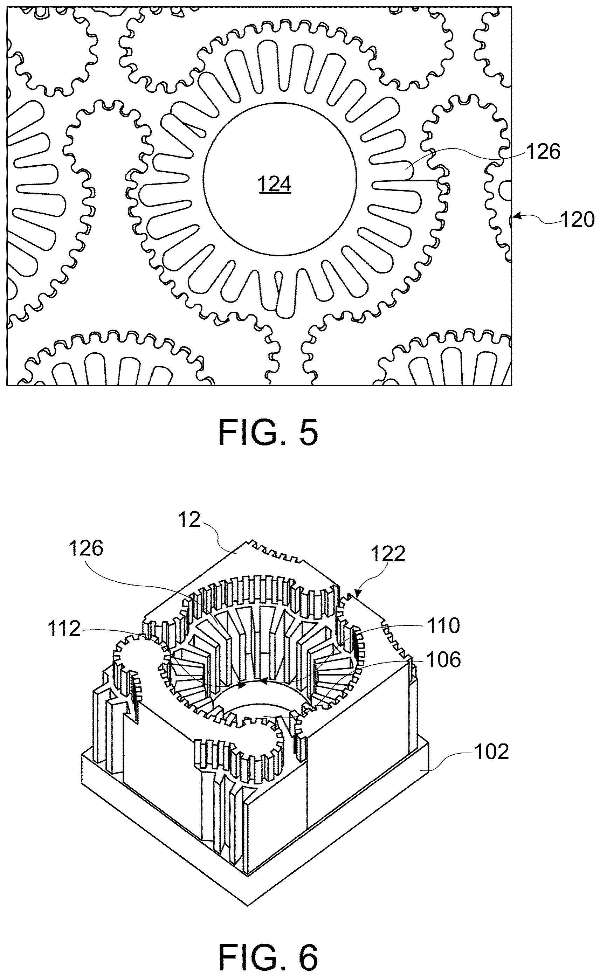

Figure 5 shows a portion of a mould formed from a master 122 having the same shape and form as the support 2 in Figure 2(a) and 2(b). It can be seen that the compartment 10 results in a corresponding protrusion 124 feature on the mould.

In a third step, as shown in Figure 4(c), the mould 120 having been filled with polymer 118 and evenly covered, such that no portion of the mould is exposed, is aligned with the substrate 102 covered with polymer 118. The alignment brings the mould 120 and substrate 102 together such that protrusions 124 align and correspond to the electrodes 6.

In a fourth step, when the mould 120 and substrate 102 are brought together under pressure the protrusion 124 squeezes excess polymer from the surface of the electrode. The elastomeric properties of the mould 120 allow it to conform and accommodate variations in the surface of the substrate 102, such as dome-shaped electrodes, uneven surface features or curvature of the surface of the substrate 104. In a fifth step, the polymer 118 is cured before the mould is separated from the substrate, in a sixth step, leaving the polymer attached to the substrate 102. The polymer has become the well array structure.

Protrusions 124 on the mould 120 have fins 126, which appear as radial fingers as viewed in Figure 5 and extend along the entirety of the length or depth of the through-holes 112. In other words, the fins can be described as open-ended, with fins being open and unconnected from the aperture 106 all the way to the opposite open end of the through-hole 106 in the well array structure. It was realised, however, that in the final demould step shown in Figure 4(f) the cured polymer between the fins 126 and/or the increased polymer connection between the well array structure and the substrate resulted in an imperfect detachment. This was attributed to the high force required to detach the mould, which was greater than it should have been because too much polymer had bonded to the substrate and was made worse by the greater surface area of the fins in that region adjacent the electrode.

In Figure 6 the through-hole 112 of the well array structure, which defines the compartment 10, has two regions - a first region with fins 126 at the open end, which faces away from the substrate, and a second region having a fin base 128, which define the aperture of the well array structure and faces the substrate 102. The fin base connects all the fins 126 in the second region to form a solid cylinder around the aperture, which defines a continuous wall portion. The fin base 128 reduces the surface area of the connection between the well array structure and the substrate adjacent the aperture - so lowers the force required to separate the two after the polymer is cured.

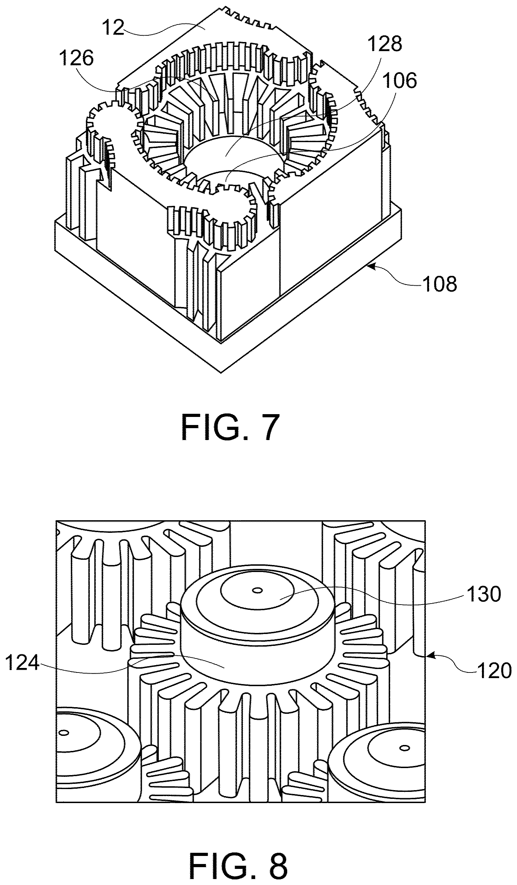

The depth of the through-hole 112 can be described with respect to Figure 6, but the description applies equally to other examples shown in the Figures. The depth is the shortest distance between the top of the fins 126 and the substrate. In Figure 6 the depth of the fin base 128 is approximately one third of the depth of the through-hole, with the fins 126 extending two thirds of that depth. Figure 7 shows a compartment 10 having a through-hole in which the fins are shorter than those shown in Figure 6 and being about one third of the depth of the through-hole.

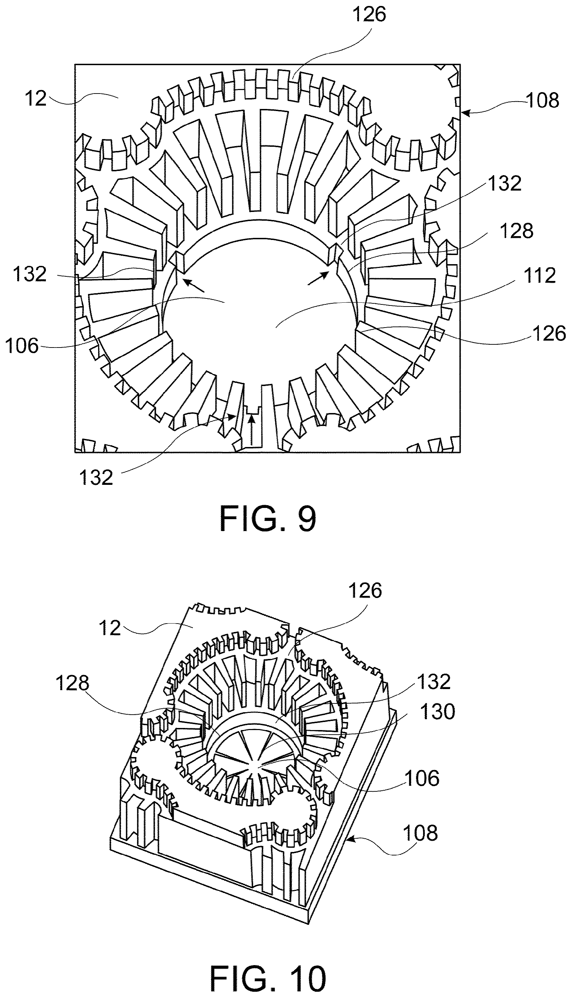

Figures 8, 11 and 12 show the shorter fins 126 on the mould 120, while Figures 9 and 10 show the resulting compartment 10 of the well array structure, wherein it can be seen that the protrusion 124 extends beyond the ends of the fins. As a consequence, the absence of the mould in this area allows the polymer to create the second region in which a continuous surface is formed around the aperture at the base of the through-hole.

The purpose of the well array structure is clearly documented in WO2014/064443. What cannot be appreciated from the known art is the moulding techniques and adaptations of the hardware required to optimise the manufacturing process and subsequent performance of a nanopore sensing device having an improved well array structure.

To this end, the mould 120 protrusion 124 can be provided with one or more extensions 130 that extend from the protrusion of the mould that creates the through-hole and clears polymer from the electrode. Each extension controls the shape or presence of the polymer after moulding by, for example, adding a recess or formation.

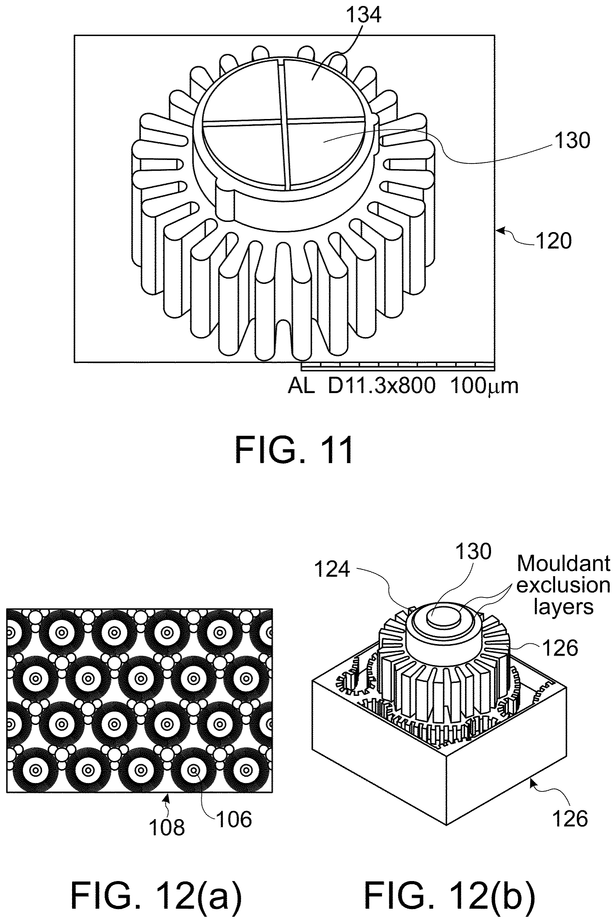

One of the original functions of the fins 126 was to retain and control the distribution of fluids within the compartment 10 and, in particular, to control the distribution of pre-treatment oil prior to the formation of membranes over the well array structure. In Figure 9, three recesses 132 are shown. In this example the recesses are shown as rectangular notches or grooves. They can be evenly spaced but could be any number or arrangement of recesses or pockets for retaining a pre-treatment oil or the like. The recesses function to inhibit oil pooling at the bottom of the compartment 10 and covering an electrode 6. Figure 11 shows three semi-cylindrical extensions 130 upon the side of the protrusion 124 that defines the through-hole and, postmoulding, form recesses in the through-holes of the well array structures.

As described above, the protrusion 124 functions to form the through-hole 112 of each compartment 10 and displace polymer from the electrode 6. Partial fouling of flat electrodes can occur and result in a film of moulded material remaining covering a portion of the electrode surface. An example of this is shown in Figure 12(a), in which Newton's rings indicate that the film of mouldant remaining is thicker in the centre. To counter this, the protrusion 124 is provided with an extension that can be curved or domed at the end of the protrusion, as shown in Figure 8, or a stepped extension of progressively smaller discs as shown in Figure 12(b). The purpose of these extensions 130 at the end of the protrusion on the mould 120 is to generate a pressure gradient on the polymer, which is a liquid moulding material, as the elastomer of the mould is compressed. This functions to extruding the liquid as the mould conforms to the electrode surface and contacts the electrode. In Figure 8, a dimple resides in the centre due to a photolithography defect. This defect turned out to allow mouldant to accumulate in the dimple while clearing surrounding areas of the electrode. One or more dimples can be provided on the top of the protrusion for the purpose of allowing mouldant to accumulate in the dimple. The dimple can, for example, be star-shaped e.g. a 5-sided star.

Extensions on the end face of the protrusion can be provided to facilitate the displacement of the polymer. By way of example, Figure 8 shows a domed extension and Figure 12 shows a tiered dome that progressively increased the pressure applied to the polymer between the protraction and the electrode when the mould aligned and brought under pressure against the substrate. By way of another example, Figure 10 shows a through-hole 112 having closed off spokes that extend from the edge of the well array structure and extend towards the centre without touching. The extensions 130 for creating such spokes can be appreciated from Figure 11, in which four segments would create 4 spokes that connected in the middle. The extensions 130 on the face of the protrusion 124 can function to displace polymer from the electrode 6 and/or be arranged to create channels 134 between the extensions 130 to provide a route for polymer to flow from the electrode when under pressure during assembly.

To mould a well array structure a mouldant 118 can be applied to the surface of a mould 120 and/or a substrate 102 to form a sensing device, and example of which is shown in Figures 4(a) and 4(b). Alternatively, the mould can be placed against the substrate and a mouldant can be injected between the mould and substrate before being processed to cure and secure the mouldant to the substrate. The mould could then be removed to leave the well array structure upon the substrate.

The mouldant 118 has been described as a polymer such as poly(methyl methacrylate). The polymer can be dissolved in a solvent before being deposited onto the underside of the mould 120. For example, PS (35KDa) can be dissolved in 500mg/mL of chloroform. After moulding the polymer can be heated to evaporate the solvent before removing the mould from the substrate.

The amount of solvent in the mouldant can be selected to inhibit any change in the dimensions of the mould 120, when susceptible to absorbing some of the solvent and losing a degree of structural rigidity.

The mould can be a thermoplastic, or it can also be made of a silicone material, such as polydimethylsiloxane (PDMS). The master 122 can be made from a robust dimensionally stable material, such as silicon wafer.

The mouldant can be a two-component thermal cure epoxy or UV curable material. Thermal cure epoxy was found to be slower to cure than UV curable epoxy and require the mould to be held in place for a longer period of time.

Mouldant materials can include one or more of epoxy, acrylamide/methacrylamide, polyester, styrene copolymer, vinyl, polyurethanes (isocyanate/amine chemistry) or polyimide.

Numerous mouldant materials are available for various manufacturers, and include: DYMAX (RTM) materials, including part 6-621, 431, 3069, 3069-GEL, 3099 and 1072-M; LOCTITE materials, including part AA 3321 LC, AA 3936, 3922, 3921, 3311, 3301 and 3211; EPOXIES materials, including part 7108, 7156 and 7159; NORLAND PRODUCTS INCORPORATED materials, including part NOA81, NOA61, NOA88 and NOA 83H; MasterBond materials, including part UV10MED, UV22DC80-1MED, UV10, UV10TKLO-2, UV16 and UV15LV; Epoxy Technology materials, including part OG198-54, MED-OG198-54, OG142-87 and OG-112; Permabond materials, including part 4UV80 and UV605; and Ostemer materials, including part '322'. By way of example, two suitable materials were from Masterbond (RTM) part UN10MED and EpoTek (RTM) part OG198-54. The connection between the well array structure 108 and the substrate can be semi-permanent. In other words, they can be peeled apart such that a formed well array structure can be removed and reapplied to another substrate. Or, after removal, a new well array structure can be formed in its place. In this way the substrate and associated devices or components can be recycled.

Although the above processes describe forming or attaching a well array structure to a substrate such that the electrodes are accessible through the through-holes 112, the electrodes 6 can be subjected to further processing to clean their surface. The cleaning can comprise at least one of chemical cleaning or plasma treatment.

The mould 120 of the invention can be independently formed on a known TSV 4, as shown in Figures 1(a) and 1(b), which would minimise the effects of processing on the surface energy of a well array structure 108, or the mould can be independently formed on a substrate such as a printed circuit board 8.

In a further example of the invention an improved substrate 102 can use alternative materials and manufacturing methods to those described above. This can enable a cost-efficient consumable, high throughput and low material cost sensor to be fabricated. This example provides an integrated electrode using laser machining and screen-printing methods. The method is compatible with reel to reel or panel to panel processing.

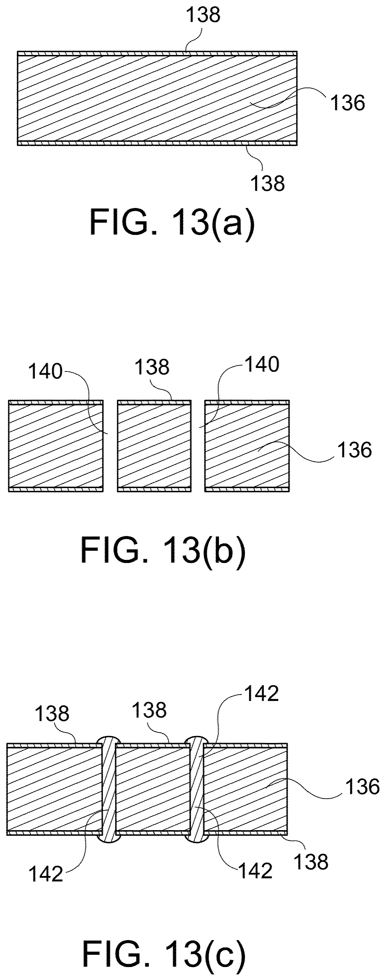

By way of example, Figures 13(a) to (e) illustrate progressive steps that can be taken to provide a substrate 102 with electrodes 6 upon which a well array structure 108 can be formed. The method involves taking a sheet 136 of thermoplastic foil that can be, for example, polyimide, polyetherimide, polyether- ether- ketone (PEEK), polycarbonate or polyethylene terephthalate (PET). The thickness is about 0.5mm or less. A metal film 138 is formed on the top and bottom side, as viewed, using a metallisation process. The metallisation process can be sputtering, and the metal applied can be, for example, silver, gold or platinum. The metallisation provides for redistribution lines (RDLs) to be formed on the surface of the sheet.

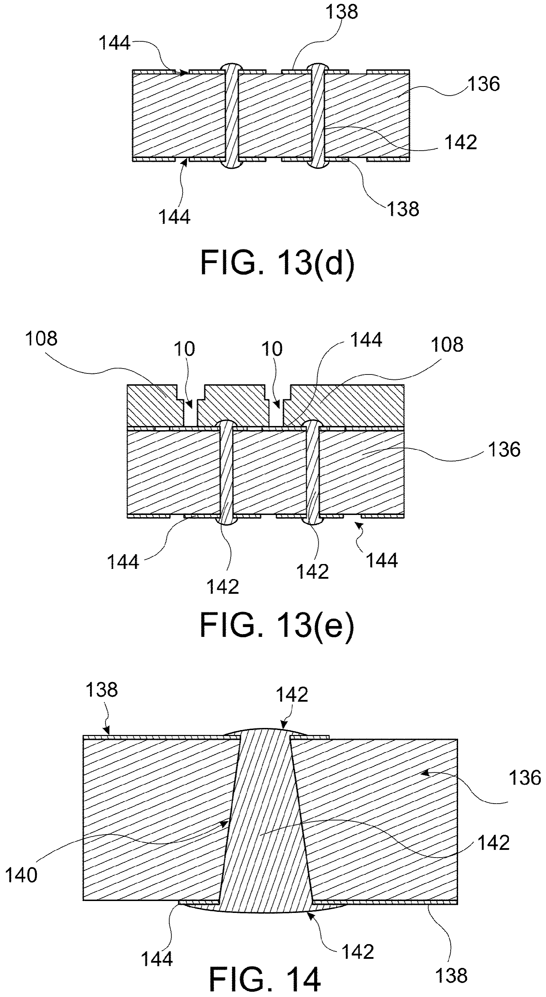

Figure 13(a) shows such a sheet 136 having a metal film 138. A laser is used to machine a plurality of vias 140 through the metalised film, as shown in Figure 13(b). The vias 140 are then filled with a conductive paste, such as carbon or silver paste, before being cured to provide conductive vias 142. Each conductive via 142 has a low resistance of between about 200 and about 300 ohms. The paste can be filled using screen-printing. When screen printed the contact between the conductive via 142 and the metal film 138 is increased by the formation of domed mushroom-like areas of excessive paste at the via 140 openings, as shown in Figure 13(c). This overflow can function to provide a connection between the metal film on each side of the sheet 136. Thereafter, and RDL pattern is formed on the metal film 138 on the top and bottom surfaces to provide a circuit having a feature 144 such as a contact track 144, tracks 144 or contact pad 144, including electrodes 6, on one or both sides, as shown in Figure 13(d). Figure 13(e) shows how a portion of the metal film 138 can be retained to define the electrode 6 and close the aperture 106 and define the bottom of a compartment 10 of a well array structure 108.

The well array structure 108, as described above, can be formed independently using (i) a photoresist layer, with or without a separate adhesive layer, or (ii) a mould 120. The use of a mould accommodates the uneven surface caused by the tracks 144 and/or the domed ends of the conductive via 142 and is particularly suited to accommodating variations in height on the surface of a substrate 102.

To implement the metal film 138, a sheet 136 can be formed by sputtering metal on to a sheet before subsequently using laser ablation to define electrodes 6 and any circuitry. By way of example, to provide a high purity electrode 6 platinum can be sputtered on to a PEEK sheet. A high-quality electrode can be provided to maximise electron transfer when the sensor device 100 is operating.

Figure 14 illustrates the impact of the shape of the via 142 upon the dimensions of the interface with the electrode, or other circuitry, of the metal film 138. It is clear that the size of the aperture of the via formed at the surface of the sheet 136 by laser drilling influences the size of the conductive via contact area. The vias formed in Figure 13(b) have parallel sides and high-aspect ratios that create small apertures in the surface and, subsequently, have minimal exposure on the surface of the sheet 136. When filled with screen-printing small dome-shaped contacts are formed. In practice, the shape of the via 140 is determined by the thickness of the sheet 136 and the drill geometry i.e. the laser drilling process. To maximise the utilisation of the surface of the sheet 136 the footprint of the via 140 and conductive via 142 should be minimised.

As an alternative to coating a sheet 136 with metalised film 138, drilling and then filling the formed via 140 with conductive paste a bare sheet without a metalised surface can be drilled first and then coated using physical vapour deposition (PVD) process. A PVD metal coating can be formed to extend from the planar surfaces of the sheet and in to the via 140 thus forming an alternative conductive via 142 to the one shown in Figure 14.

In practice, a high-aspect ratio via extending perpendicularly from one side of the sheet to the other, as shown in Figure 13(b) has a very small aperture on the surface and a taper angle of 90 degrees. Consequently, PVD coating the walls of the via furthest from the surface of the sheet is difficult because access is restricted.

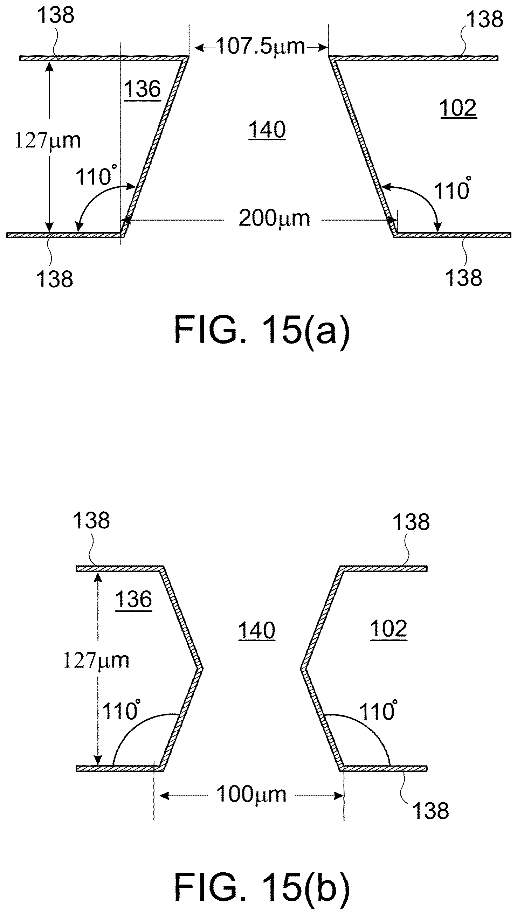

Figure 15(a) shows the dimensions of a sheet drilled from one side and the metal coating 138 coating that forms a via with flat sides, while Figure 15(b) shows a sheet drilled from both sides, and the subsequently applied PVD coating 138 that forms a via with ridged or hipped sides. The angle between the planar surface of the sheet and the side of the via extending therefrom is the taper angle. Therefore, the taper angle at one or both sides is greater than 90 degrees.

When the taper angle is greater than 90 degrees on only one side of the sheet 136, as shown in Figure 15(a), one opening of the via is greater in size than the other. In this case the ratio of aperture size or diameter of one side to the other can be about 1:4, or preferably about 1:3 and more preferably about 1:2. In this arrangement one side has an aperture receptive to PVD such that the walls of the via are coated all the way through.

Drilling from both sides results in the taper angle being greater than 90 degrees on both sides of the sheet 136, as shown in Figure 15(b). The openings can have different sizes, although the via can be substantially symmetrical - appearing to have two lines of symmetry, one horizontal and one vertical, as viewed. In this case the ratio between the aperture size or diameter on one side compared to the opposite side, can be about 1:1. This minimises the footprint of the via as viewed from the surface of the sheet, and as viewed from both sides. Moreover, in the arrangement of Figure 15(b) both sides of the via are receptive to PVD such that the walls of the via are coated all the way through.

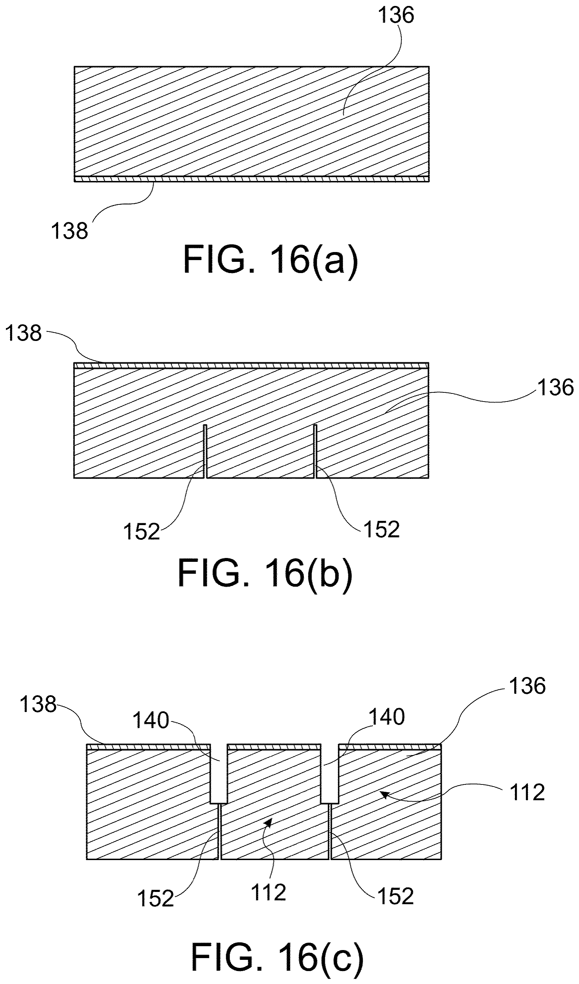

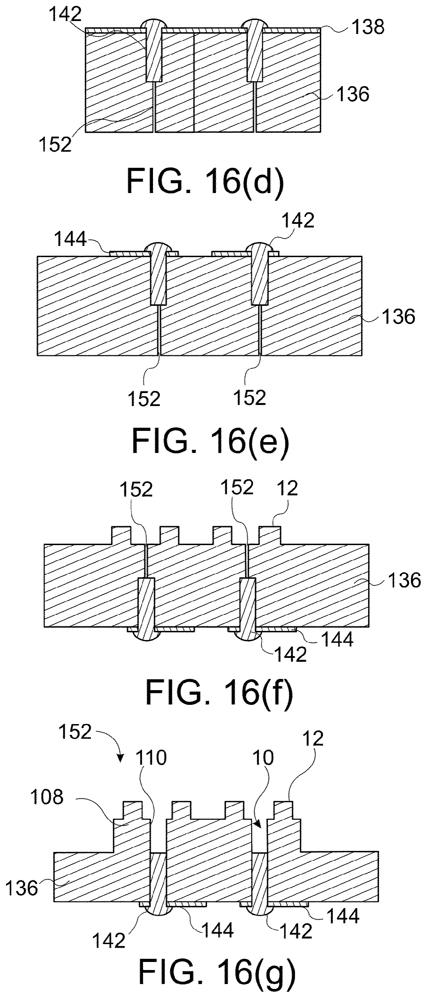

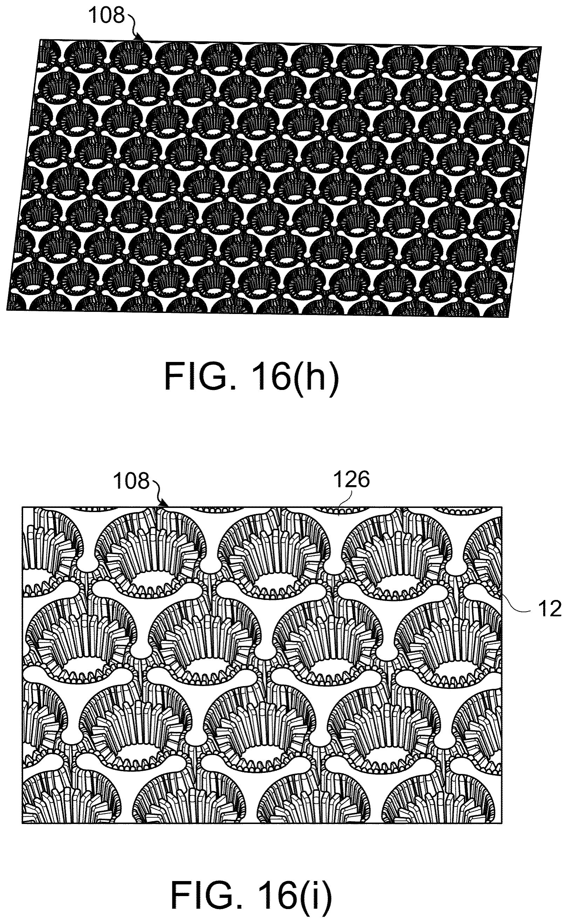

Figures 16(a) to (g) show fabrication steps for a hybrid well array 150 that incorporates the function of the substrate 102 and well array structure 108, described above, into a singlepiece component for nanopore sensing. Like reference numerals refer to like features. The fabrication of the hybrid well array 150 begins with a sheet 136 having a metal film 138 formed only on one side, or having a metal film deposited only on one side, to provide a sheet as shown in Figure 16(a). Vent holes 152 are drilled on the opposite side of the sheet to the metal film, as shown in Figure 16(b) before a partial via 140 is drilled through the metal film on the opposite side of the sheet 136 to the vent 152. The resulting structure can be seen in Figure 16(c) having a via 140 extending from one side of the sheet to the other, said via having a wide drilled portion and the narrow vent 152. In Figures 16(d) and 16(e) the wide end of the via 140 is filled with a conductive paste before the metal film is trimmed to form a circuit connected to the conductive vias 142. It is to be noted that the vent is too narrow to fill, or fill completely, with the conductive paste. The purpose of the vent is to allow the wide portion of the via to fill completely by inhibiting trapped air from preventing the via being filled. Thereafter, the vent 152 side of the sheet 136 is processed to form pillars 12 as shown in Figure 16(f) before material around those pillars is removed to define walls 110 of compartments 10 and expose the conductive via 142 as shown in Figure 16(g).

The properties of the electrodes 6 on the substrate 102 influence the performance of the sensing device 100. As described above, the sheet 136 can be sputtered with metal and subsequently laser ablated. Optimum performance has been achieved with sputtered platinum upon a poly(etheretherketone) PEEK sheet. Vias 140 can be subsequently filled. The base of the compartment 10 can be defined by the sputtered metal film 138 that is connected to a conductive via 142, as shown by way of example in Figure 13(e). The conductive vias 142 are configured to provide low resistance electrical contacts with resistance values for between 150ohms and 300ohms, or between 200ohms and 300ohms.

The material selected for the sheet 136 was PEEK. In the examples, the sheet had a dual gloss finish and a thickness of 0.25mm. PEEK was chosen based on at least one of a number of factors, including: mechanical stability to provide reliable connection with a connector, such as Samtec z-array connector (https://www.samtec.com/connectors/high-speed-board-to-board/high-density-arrays/zray); the PEEK had good adhesion properties with a sputtered platinum layer allowing high resolution track and gaps to be formed without flaking; and PEEK is suitable for achieving high aspect ratio laser drilled via holes. Other materials found to be suitable with comparable performance include Polyetherimide (PEI).

Before sputtering, the sheet 136 is cleaned with, for example, ethanol before loading into a sputtering chamber. In the examples, 50nm of platinum was coated on both sides of the film.

A laser was used to pattern and drill the metalised sheet 136. The pattern laser can be, for example, a SMI laser (MSV-301). The drilling laser can be, for example, a direct write laser (MSV-101 UV).

The vias 140 were filled was carried out using a DEK^{™} screen print process. Silver ink was used to fill the vias from the backside before flipping the panel and capping the vias on the frontside. The ink was then cured in the oven at 110°C.

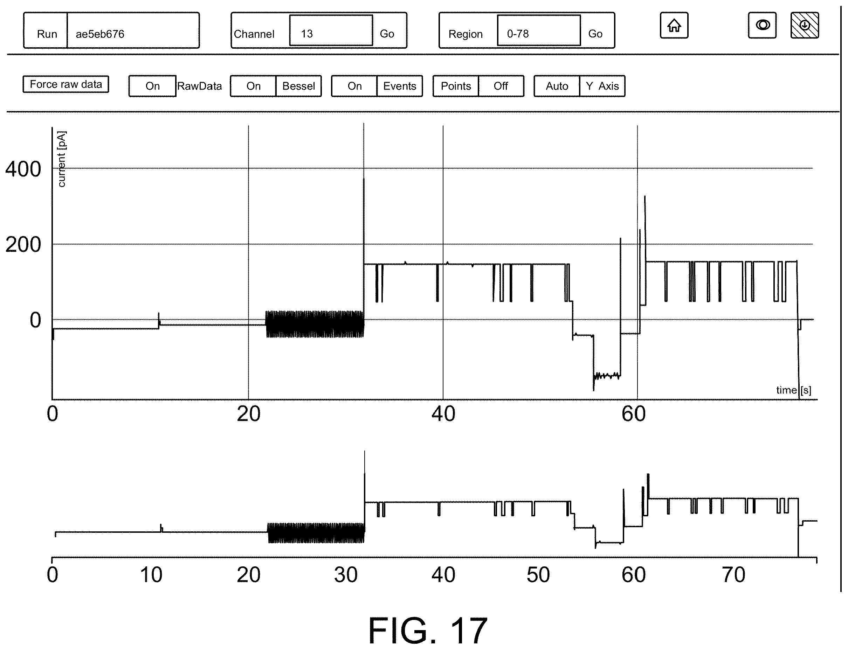

In each of the examples the performance of the sensing device 100 was evaluated by manufacturing a known MinION (RTM) device with the combined well array structure 108 and substrate 102, or hybrid well array 150, in place of known structure 2 and wafer 4. After assembly, the well array structures were fluid filled and the membranes formed across the compartments 10 were populated with biological nanopores. During the evaluation, indicators of adequate performance include: an open pore current between about 180 to 200pA with 180mV applied across the pore; noise levels of between about 2 to 3pA standard deviation on the open pore current; and sharp thrombin binding apatamer (TBA) events indicating no additional high RC time constants in the setup. Details of TBA use is described in WO2014/064443. An ion channel recording with TBA on each of the examples demonstrated results like those shown in Figure 17, which shows the results of a well array structure 108 made with photoresist upon a substrate 102 made with a plastic sheet 136, as described above.

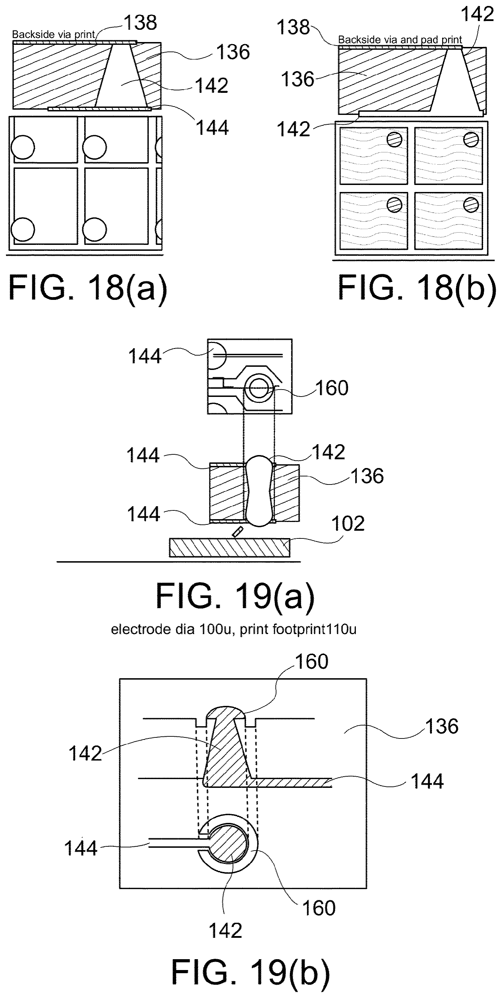

A number of examples have been described as having a sheet 136 with a metal film 138 on both sides, with tracks and/or contact pads formed in the or each metal film. As a lower cost alternative the sheet can be coated with a metal film on one side, while contact pads are screen printed on the non-metalised side, thus no metal patterning is required. The vias 140 can be filled and the contact pads formed in a single step. Figure 18(a) shows a schematic of a sheet having a metal film on both sides, with a conductive via 142 therethrough connected to a contact pad 144. The surface of both metal films 138 have been trimmed - this schematic is shown above an image of an actual pad. In the example of Figure 18(a) the metal films are platinum.

Figure 18(b) is a schematic of a sheet having a metal film on one side, and when the conductive via 142 is screen printed on the sheet 136 the contact pad 144 is formed at the same time. The conductive via 142 and contact pad 144 both have the same material, which in this example is silver.

The density of vias 142, tracks 144 or contact pads 144 formed during screen printing processes is influenced by the controlled spread of paste. The channel density is defined by a combination of the track or pad size, the gaps between and the print size. Preventing spread of the ink during printing is important to tightly define print size. This also inhibits short circuits by controlling the deposition and spread of conductive paste. A capillary stop 160 can be formed adjacent a via 140 to inhibit screen printed ink from spreading. A plan view of an conductive via 142 adjacent a track is shown in Figure 19(a) aligned with a sectional elevation view of a sheet 136 with the conductive via 142 and beneath the sheet a PCB. The diameter of the conductive via 142 forming an electrode is 100 microns, while the capillary stop is shown extending around the aperture created by the via 140, which limits the print diameter to 110 microns. Figure 19(b) shows the geometry of the capillary stops 160 in more detail. A groove is formed adjacent but separated from the aperture of the via 140 in the sheet 136. The level of the aperture is in the same plane as the sheet 136, and the groove extends into the sheet. In the example shown the groove does not enclose the via to allow a track to extend along the planar surface of the sheet 136. The groove of the capillary stop is an effective way of providing pinning points to prevent ink spreading.

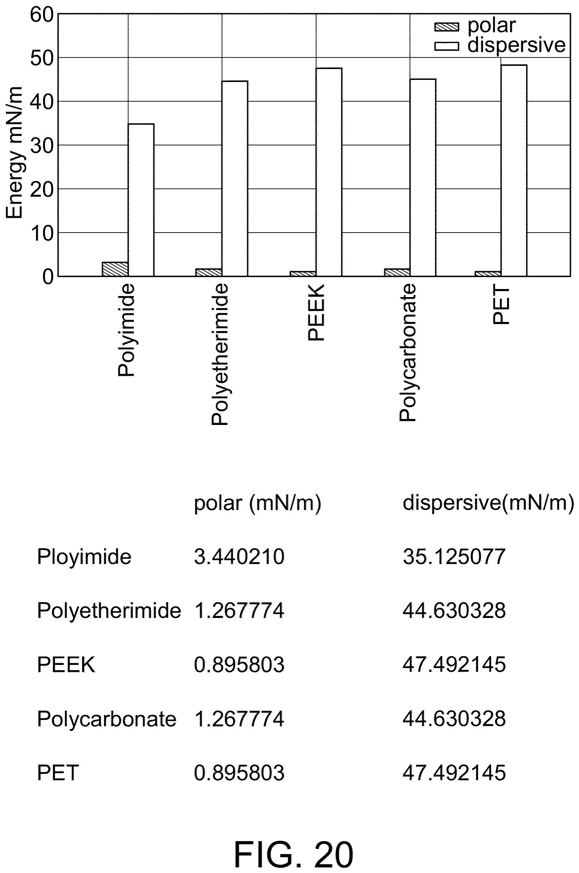

The material selected for the sheet 136 took in to account its application as a well array structure in a nanopore sensing device 100. The values required of a dispersive component of the sheet material is 40-55mN/m and the polar component should be less than <3mN/m. Contact values of PEI, PEEK, PC and PET were assessed and their properties tabled, as shown in Figure 20. During the evaluation of these materials their native surface properties through the laser ablation process is also key. These surface properties can be tuned based on atmosphere during the ablation steps minimising oxidation.

To obtain the required surface energy while not relying on maintaining the bulk properties the process can involve: functional groups remaining at the surface post ablation, which can be modified to achieve the required surface properties (silanes); and surface coating using plasma treatments / thin film depositions.

In the examples above, a well array structure 108 is attached to a substrate 102 having electrodes 6. The substrate can include one of: a printed circuit board; plastic interposer made from a laser ablated plastic sheet 136; silicon interposer; and the surface of an application specific integrated circuit (ASIC) having exposed terminals on one face thereof to function as the electrodes. The substrate 102 can be a plastic sheet 136 that has a well array structure laser ablated into the surface on one side.

The well array structure 108 can be formed of a single component, which can include one of: a moulded material, such as a moulded polymer 118; a three-dimensionally printed object; or a patterned photoresist material.

A three-dimensionally printed object can be made from material, such as dielectric ink (UV Curable Acrylate), pseudo-polyimide inks, polyetherketone, polylactic acid, dielectric ceramics e.g. an aqueous solution of Li2MoO4 and ceramic particles, polyetherimide and polyimide.

Alternatively, the well array structure 108 can include two separate layers, as described and shown in relation to Figure 2(a) above. That is, the well array structure can have a base layer 108a that is analogous to the support 2 shown in Figure 2(a), and a support layer 108b that is analogous to the layer having pillars 12 extending perpendicularly from the surfaces adjacent the well 10. The support layer 108b, therefore, can be said to extend at another level or layer from the support 2 in the well array structure 108.

The base layer 108a can be configured as a permanent layer in the device 100, and the support layer 108b can be removably attached therefrom to enable the device to be processed for re-use.

The base layer 108a can include at least one of: a moulded polymer 118; a three-dimensionally printed layer; plastic interposer made from a laser ablated plastic sheet 136; a patterned photoresist material; glass; silicon or silicon dioxide. The base layer has an array of walls 110 defining through-holes 112 with the apertures 106 for defining the wells 10 when connected to the substrate 102.

The support layer 108b can include at least one of: a moulded polymer 118; a three-dimensionally printed layer; plastic interposer made from a laser ablated plastic sheet 136; a patterned photoresist material; glass; or silicon dioxide. The support layer has pillars 12 that support the retention of oil for forming amphiphilic membranes across the wells 10. Alternatively, the support layer can comprise solid-state nanopores or hybrid nanopores (biological nanopores located in solid-state apertures).

In light of the teaching herein, various combinations of substrate 102, base layer 108a and support layer 108b are configurable.

The well array structure, or hybrid well array, of the sensing device of the examples, can use any membrane in accordance with various aspects described herein. Suitable membranes are well-known in the art. The membrane can be an amphiphilic layer or a solid-state layer. An amphiphilic layer is a layer formed from amphiphilic molecules, such as phospholipids, which have both hydrophilic and lipophilic properties. The amphiphilic molecules may be synthetic or naturally occurring. Non-naturally occurring amphiphiles and amphiphiles which form a monolayer are known in the art and include, for example, block copolymers (Gonzalez-Perez et al., Langmuir, 2009, 25, 10447-10450). The copolymer may be a triblock, tetrablock or pentablock copolymer. The membrane can be a triblock or diblock copolymer membrane.

Membranes formed from block copolymers hold several advantages over biological lipid membranes.

Block copolymers may also be constructed from sub-units that are not classed as lipid sub-materials; for example, a hydrophobic polymer may be made from siloxane or other nonhydrocarbon-based monomers. The hydrophilic sub-section of block copolymer can also possess low protein binding properties, which allows the creation of a membrane that is highly resistant when exposed to raw biological samples. This head group unit may also be derived from nonclassical lipid head-groups.

Blockcopolymer membranes also have increased mechanical and environmental stability compared with biological lipid membranes, for example a much higher operational temperature or pH range. The synthetic nature of the block copolymers provides a platform to customize polymer-based membranes for a wide range of applications.

The membrane and oils disclosed herein can be one of the membranes or oils disclosed in WO2014/064443 and WO2014/064444 hereby incorporated by reference in its entirety. These documents also disclose suitable polymers.

The amphiphilic molecules may be chemically modified or functionalized to facilitate coupling of the polynucleotide.

The amphiphilic layer may be a monolayer or a bilayer. The amphiphilic layer is typically planar. The amphiphilic layer may be curved. The amphiphilic layer may be supported. The amphiphilic layer may comprise one or more tethering moieties to tether analyte to the surface. The amphiphilic layer may be concave. The amphiphilic layer may be suspended from raised pillars such that the peripheral region of the amphiphilic layer (which is attached to the pillars) is higher than the amphiphilic layer region.

The membrane may be a lipid bilayer. Suitable lipid bilayers are disclosed in WO 2008/102121, WO 2009/077734 and WO 2006/100484.

Methods for forming lipid bilayers and other amphiphilic membranes are known in the art. Lipid bilayers are commonly formed by the method of Montal and Mueller (Proc. Natl. Acad. Sci. USA., 1972; 69: 3561-3566), in which a lipid monolayer is carried on aqueous solution/air interface past either side of an aperture which is perpendicular to that interface.

Solid state layers can be formed from both organic and inorganic materials including, but not limited to, microelectronic materials, insulating materials such as Si₃N₄, A1₂O₃, and SiO, organic and inorganic polymers such as polyamide, plastics such as Teflon^{®} or polymers such as two-component addition-cure silicone rubber, and glasses. The solid-state layer may be formed from graphene, or other mono-atomic layer, such as MoS2. Suitable graphene layers are disclosed in WO 2009/035647. Yusko et al., Nature Nanotechnology, 2011; 6: 253-260 and US Patent Application No. 2013/0048499 describe the delivery of proteins to transmembrane pores in solid state layers without the use of microparticles.

Any transmembrane pore may be used. The pore may be biological or artificial. Suitable pores include, but are not limited to, protein pores, polynucleotide pores and solid-state pores. The pore may be a DNA origami pore (Langecker et al., Science, 2012; 338: 932-936).

The transmembrane pore can be a transmembrane protein pore that typically permits hydrated ions, driven by an applied potential to flow from one side of the membrane to the other. The transmembrane protein pore may allow an analyte such as polynucleotide to be moved through the pore.

The transmembrane protein pore may be a monomer or an oligomer. The pore can be made up of several repeating subunits, such as at least 6, at least 7, at least 8, at least 9, at least 10, at least 11, at least 12, at least 13, at least 14, at least 15, or at least 16 subunits. The pore
can be a hexameric, heptameric, octameric or nonameric pore. The pore may be a homo-oligomer or a hetero-oligomer.

The transmembrane protein pore typically comprises a barrel or channel through which the ions may flow. The subunits of the pore typically surround a central axis and contribute strands to a transmembrane β barrel or channel or a transmembrane α-helix bundle or channel. The barrel or channel of the transmembrane protein pore typically comprises amino acids that facilitate interaction with nucleotides, polynucleotides or nucleic acids. These amino acids can be located near a constriction of the barrel or channel. The transmembrane protein pore typically comprises one or more positively charged amino acids, such as arginine, lysine or histidine, or aromatic amino acids, such as tyrosine or tryptophan. These amino acids typically facilitate the interaction between the pore and nucleotides, polynucleotides or nucleic acids.

Transmembrane protein pores for use in accordance with the invention can be derived from β-barrel pores or α-helix bundle pores. The transmembrane pore may be derived from or based on Msp, α-hemolysin (α-HL), lysenin, CsgG, ClyA, Sp1 and hemolytic protein fragaceatoxin C (FraC). The transmembrane protein pore can be derived from CsgG. Suitable pores derived from CsgG are disclosed in WO 2016/034591. The transmembrane pore may be derived from lysenin. Suitable pores derived from lysenin are disclosed in WO 2013/153359.

The analytes (including, *e.g.,* proteins, peptides, small molecules, polypeptide, polynucleotides) may be present in an analyte. The analyte may be any suitable sample. The analyte may be a biological sample. Any example of the methods described herein may be carried out *in vitro* on an analyte obtained from or extracted from any organism or microorganism. The organism or microorganism is typically archaean, prokaryotic or eukaryotic and typically belongs to one of the five kingdoms: plantae, animalia, fungi, monera and protista. In some examples, the methods of various aspects described herein may be carried out *in vitro* on an analyte obtained from or extracted from any virus.

The analyte can be a fluid sample derived from any source such as biological, industrial or environmental. The analyte can comprise a body fluid. The body fluid may be obtained from a human or animal. The human or animal may have, be suspected of having or be at risk of a disease. The analyte may be urine, lymph, saliva, mucus, seminal fluid or amniotic fluid, but can be whole blood, plasma or serum. Typically, the analyte is human in origin, but alternatively it may be from another mammal such as from commercially farmed animals such as horses, cattle, sheep or pigs or may alternatively be pets such as cats or dogs. Alternatively, an analyte can be of plant origin.

The analyte may be a non-biological sample. The non-biological sample can be a fluid sample. An ionic salt such as potassium chloride may be added to the sample to effect ion flow through the nanopore. The polynucleotide may be single stranded or double stranded. At least a portion of the polynucleotide may be double stranded.

The polynucleotide can be a nucleic acid, such as deoxyribonucleic acid (DNA) or ribonucleic acid (RNA). The polynucleotide can comprise one strand of RNA hybridised to one strand of DNA. The polynucleotide may be any synthetic nucleic acid known in the art, such as peptide nucleic acid (PNA), glycerol nucleic acid (GNA), threose nucleic acid (TNA), locked nucleic acid (LNA) or other synthetic polymers with nucleotide side chains. The polynucleotide can be any length.

Any number of polynucleotides can be investigated. For instance, the method may concern characterising 2, 3, 4, 5, 6, 7, 8, 9, 10, 20, 30, 50, 100 or more polynucleotides. If two or more polynucleotides are characterised, they may be different polynucleotides or two instances of the same polynucleotide. The polynucleotide can be naturally occurring or artificial.

The method may involve measuring two, three, four or five or more characteristics of a polynucleotide. The one or more characteristics can be selected from (i) the length of the polynucleotide, (ii) the identity of the polynucleotide, (iii) the sequence of the polynucleotide, (iv) the secondary structure of the polynucleotide and (v) whether or not the polynucleotide is modified.

The nanopore may be part of an array comprising a plurality of nanopores such as disclosed in WO2014064443. The polynucleotides may be caused to translocate the array of nanopores and a parameter such as current measured over time from which the sequence of the polynucleotide may be determined, such as disclosed in WO 2013041878 and WO2014135838. Suitable electrical measurements, are described in Stoddart D et al., Proc Natl Acad Sci, 12;106(19):7702-7, Lieberman KR et al, J Am Chem Soc. 2010;132(50):17961-72, and International Application WO 2000/28312. Other types of measurements may be carried out for example optical measurements such as fluorescence measurements and FET measurements. Optical measurements and electrical measurements may be carried out simultaneously, see for example Heron AJ et al., J Am Chem Soc. 2009; 131(5): 1652-3).

The polynucleotide may be labelled, and measurements may be carried out to determine the polynucleotide sequence from measurement of the labels. The analyte may be an expandomer such as disclosed by WO2014/074922.

The secondary structure may be measured in a variety of ways. For instance, if the method involves an electrical measurement, the secondary structure may be measured using a change in dwell time or a change in ion current flowing through the pore. This allows regions of single-stranded and double-stranded polynucleotide to be distinguished.

### CLAUSES

1. A method of forming a sensing device for supporting a plurality of nanopores upon an array of wells, the method comprising:
   providing a substrate, said substrate having a surface having an array of electrodes located thereon for connecting to or for configuring upon an electronic circuit,
   separately providing a well array structure, having an array of walls defining through-holes for defining wells, and
   aligning said through-holes and said array of electrodes such that said well array structure becomes part of the substrate and such that said electrodes define, at least in part, a portion of the bases of respective wells.
2. A method as recited in clause 1, further comprising providing an electronic circuit formed independently of said well array structure which contacts or comprises said electrodes.
3. The method as recited in clause 2 wherein said electronic circuit is provided on said substrate and said method comprises forming said well array structure onto said substrate.
4. The method as recited in clause 3 comprising moulding the well array structure onto the substrate.
5. The method as recited in clause 1 or 2 comprising forming said through-holes in said substrate and forming said electrodes by providing a conductive material in each through- hole to form at least part of a base of a respective well, or connect to an electrode in the base of the well.
6. The method as recited in clause 5 wherein said electronic circuit is provided on a further substrate and said method comprises attaching said further substrate to said sensing device such that said electronic circuit is electrically connected to said electrodes.
7. The method as recited in clause providing said well array structure comprises attaching a pre-formed well array structure to said substrate wherein the electronic circuit is provided on a printed circuit board.
8. The method as recited in any preceding clause wherein the well array structure has a thickness of less than 0.5mm.
9. The method as recited in any preceding clause wherein the wells in the well array structure have a pitch of between about 10 and about 1000 microns, and preferably between about 200 and about 800 microns, and more preferably between about 150 and about 250 microns.
10. The method as recited in any preceding clause wherein the through-holes have a diameter of less than 200 microns.
11. A method of forming a sensing device for supporting a plurality of nanopores upon an array of wells, the method comprising:
   providing a substrate having an array of electrodes located on a surface thereof for connecting to or forming part of an electronic circuit,
   providing a mould defining an inverse well array structure comprising an array of protrusions on an underside thereof;
   depositing a polymer onto the underside of the mould;
   aligning the underside of the mould that is filled with polymer against the substrate such that the protrusions are each aligned with a corresponding electrode;
   applying a mutual pressure to the substrate and the protrusions to exclude the polymer from at least a portion of the electrodes;
   processing the polymer to cure and secure the polymer to the substrate; and
   separating the mould from the polymer, whereby an array of wells of the well array structure are formed by through-holes in the polymer corresponding to the protrusions and a single electrode forming at least part of a base of each well.
12. The method as recited in clause 11 wherein the protrusions each define in respective through-holes an array of fins terminated by a continuous wall portion adjacent said substrate formed by a distal portion of said protrusion having a continuous perimeter.
13. The method as recited in clause 11 or 12 wherein providing the mould comprises depositing a moulding material onto a master well array structure comprising a plurality of wells to form the mould; and separating the mould from the master well array structure.
14. The method as recited in clause 12, wherein the moulding material is a thermoplastic.
15. The method as recited in clause 13 or 14, wherein the moulding material is polydimethylsiloxane.
16. A method as recited in clause 13, 14 or 15, wherein the master well array structure is fabricated from a silicon wafer.
17. The method as recited in any of clauses 11 to 16, wherein the polymer is poly(methyl methacrylate).
18. The method as recited in any of clauses 11 to 17, wherein the polymer is dissolved in a solvent before being deposited onto the underside of the mould.
19. The method as recited in clause 18, comprising heating the polymer to evaporate the solvent.
20. A method as recited in any of clauses 11 to 19, wherein before positioning the underside of the mould against the substrate, a polymer is deposited onto the substrate.
21. A method as recited in any of clauses 11 to 20, wherein the cured polymer is subsequently peelable from the substrate.
22. A mould adapted for use in the method described in clause 11 or 12.
23. A master well array structure, adapted for use in the method as recited in clause 13.
24. A method of forming a sensing device for supporting a plurality of nanopores upon an array of wells, the method comprising:
   providing a well array structure having an array of walls defining through-holes for forming wells
   providing a substrate having an array of electrodes located on a surface thereof for connecting to or forming part of an electronic circuit,
   providing a patterned adhesive surface on at least one of the well array structure or the substrate, wherein said patterned adhesive surface defines a plurality of portions without adhesive;
   thereafter, aligning the well array structure and substrate such that the electrodes are aligned with the plurality of portions without adhesive and with the through-holes in the well array structure such that the electrodes define, at least in part, a portion of a single well;
   using the patterned adhesive surface to adhere the well array structure to the substrate.
25. A method as recited in clause 24 further comprising providing an electronic circuit formed independently of said well array structure which contacts or comprises said electrodes
26. The method as recited in clause 24 or 25, comprising heating the adhesive surface to adhere the well array structure to the substrate.
27. The method as recited in clause 24, 25 or 26, comprising exposing the adhesive surface to electromagnetic radiation to adhere the well array structure to the substrate.
28. The method as recited in any of clauses 24 to 27, comprising heating the adhesive surface only to a temperature below the outgassing temperature of components of the electronic circuit.
29. The method as recited in any of clauses 24 to 28 wherein providing the patterned adhesive surface comprises exposing a photo-patternable adhesive to light.
30. The method of as recited in clause 24, wherein exposing the photo-patternable adhesive to light provides said plurality of portions without adhesive.
31. The method as recited in any of clauses 20 to 26, wherein the adhesive surface is integrated into one of the well array structure or the substrate.
32. The method as recited in any one of clauses 24 to 30 further comprising applying the patterned adhesive surface as an independent layer to the well array structure or the substrate.
33. The method as recited in clause 32, comprising providing an adhesive layer on a liner and forming a pattern in said adhesive layer, thereby forming said independent patterned adhesive surface layer.
30. The method as recited in clause 28, further comprising transferring the independent layer onto the well array structure or the substrate using an intermediate surface.
31. The method as recited in clause 30, wherein the intermediate surface is formed from polydimethylsiloxane.
32. The method as recited in clause 28, further comprising providing the patterned adhesive surface layer on the well array structure or the substrate using an aerosol spray or micro-drop dispenser.
33. The method as recited in clause 28, comprising adhering the well array structure to the substrate using capillary action of an adhesive to form said patterned adhesive surface layer.
34. A method of forming a sensing device for supporting a plurality of nanopores upon an array of wells, the method comprising
   providing a substrate having an electrically conductive layer on a backside thereof;
   forming vias penetrating through the substrate;
   at least partially filling said vias with a conductive filling material to form electrodes;
   removing a portion of the electrically conductive layer to form electrical contacts each in contact with a respective electrode for connecting to an electronic circuit;
   removing part of the frontside of said substrate to form an array of wells, such that each electrode forms at least in part, a portion of a single well.
35. A method as recited in clause 34, further comprising providing an electronic circuit formed independently of said well array structure which contacts said electrodes.
36. A method as recited in clause 34 or 35 comprising applying a coating to said conductive filling material to provide said electrode.
37. A method as recited in clause 34, 35 or 36, wherein removing part of the frontside of said substrate comprises ablating the substrate.
38. A method as recited in any of clauses 34 to 37, comprising using a laser to remove part of the frontside of said substrate.
39. A method as recited in any of clauses 34 to 38, comprising using scanned mask imaging to form the well array structure.
40. A method as recited in any of clauses 34 to 39, comprising forming said vias in first and second steps wherein the first step comprises forming a first part of the via and the second step comprises forming a second part of the via having a greater cross-sectional area than the first part of the via.
41. A method as recited in clause 40, comprising filling only the second part of the via with the conductive filling material.
42. A method of forming a well array device as recited in any of clauses 34 to 41, wherein forming said well array structure comprises initially forming a pillar layer and subsequently forming a layer of wells between respective pillars.
43. A method of forming a well array device as recited in any of clauses 34 to 42, wherein partially filling said vias with said conductive filling material comprises forming the conductive filling material into protrusions on the backside of the thermoplastic substrate.
44. A method of forming a sensing device for supporting a plurality of nanopores upon an array of wells, the method comprising
   providing a substrate having an electrically conductive layer on at least a frontside thereof;
   forming vias penetrating through the substrate;
   filling said vias with a conductive filling material;
   removing a portion of the electrically conductive layer to form a plurality of electrodes on the frontside of said substrate
   attaching to the frontside of the substrate a well array structure comprising an array of walls defining through-holes which together with the electrodes define a plurality of wells, such that each electrode forms at least in part, a portion of a single well.
45. A method as recited in clause 44, further comprising providing an electronic circuit formed independently of said well array structure on a backside of the substrate wherein said electronic circuit is electrically connected to said electrodes by said vias.
46. A method as recited in clause 44, wherein said substrate has an electrically conductive layer on a backside thereof, the method further comprising removing a portion of the backside electrically conductive layer to form electrical contacts each in electrical contact with a respective electrode for connecting to an electronic circuit.
47. A method as recited in clause 46 comprising providing an electronic circuit formed independently of said well array structure and connecting said electronic circuit to said electrical contacts on said substrate.
48. A method as recited in any of clauses 44 to 47, wherein attaching the well array structure onto the frontside of the substrate comprises:
   providing a patterned adhesive surface on at least one of the well array structure or the substrate, wherein said patterned adhesive surface defines a plurality of portions without adhesive;
   thereafter, aligning the well array structure and substrate such that the electrodes are aligned with the plurality of portions without adhesive and with the through-holes in the well array structure such that the electrodes define, at least in part, a portion of a single well;
   using the patterned adhesive surface to adhere the well array structure to the substrate.
49. A method of as recited in any of clauses 44 to 47, wherein attaching the well array structure onto the frontside of the thermoplastic substrate comprises:
   providing a mould defining an inverse well array structure comprising an array of protrusions on an underside thereof;
   depositing a polymer onto the underside of the mould;
   aligning the underside of the mould that is filled with polymer against the substrate such that the protrusions are each aligned with a corresponding electrode; applying a mutual pressure to the substrate and the protrusions to exclude the polymer from at least a portion of the electrodes;
   processing the polymer to cure and secure the polymer to the substrate; and
   separating the mould from the polymer, whereby an array of wells of the well array structure are formed by through-holes in the polymer corresponding to the protrusions and a single electrode forming at least part of a base of each well.
50. A method as recited in any of clauses 44 to 49, wherein filling said vias with said conductive filling material comprises forming the conductive filling material into protrusions on the frontside and/or the backside of the substrate.
51. A method as recited in any of clauses 34 to 50, wherein the conductive filing material comprises carbon and/or silver paste.
52. A method as recited in any of clauses 34 to 51, wherein the electrically conductive layer comprises platinum and/or silver.
53. A method as recited in any preceding clause comprising adding an aqueous solution to the wells of the well array structure.
54. A method as recited in clause 50 wherein the aqueous solution comprises a polar medium.
55. A method as recited in clause 53 or 54 further comprising forming a plurality of membranes comprising amphiphilic molecules in the wells of the well array structure to encapsulate the aqueous solution therein, thereby forming an array of discrete electrically isolated volumes.
56. A method as recited in clause 55 wherein the amphiphilic molecules comprise a hydrophilic head group and a hydrophobic tail group.
57. A method as recited in any preceding clause, wherein the substrate is a thermoplastic substrate.
58. A method as recited in any preceding clause, wherein the substrate initially has a uniform thickness.
59. A sensor apparatus comprising:
   an electronic circuit;
   a substrate;
   an array of electrodes located on a surface of the substrate;
   a well array structure, moulded from a polymer, comprising a plurality of walls defining through-holes which together with the electrodes define a plurality of wells, such that each 10 electrode forms at least in part, a portion of a single well; and
   wherein the array of electrodes form part of the electronic circuit or are connected to the electronic circuit.
60. A sensor apparatus as recited in clause 60 wherein the polymer is poly(methyl 15 methacrylate) (PMMA).
61. The apparatus as recited in clause 59 wherein the wells each define an array of fins terminated by a continuous wall portion adjacent said substrate.
62. A sensor apparatus comprising:
   an electronic circuit;
   a substrate;
   an array of electrodes located on a surface of the substrate;
   a well array structure comprising a plurality of walls defining through-holes which together with the electrodes define a plurality of wells, such that each electrode forms at least in part, a portion of a single well, wherein said wells each comprise an array of fins terminated by a continuous wall portion adjacent said substrate; and
   wherein the array of electrodes form part of the electronic circuit or are connected to the electronic circuit.
63. A sensor apparatus comprising:
   an electronic circuit;
   a thermoplastic substrate;
   an array of electrodes located on a surface of the substrate;
   a well array structure comprising a plurality of walls defining through-holes which together with the electrodes define a plurality of wells, such that each electrode forms at least in part, a portion of a single well; and
   wherein the array of electrodes is connected to the electronic circuit.
64. A sensor apparatus comprising:
   a substrate comprising a plurality of vias at least partially filled with conductive filing material thereby forming an array of electrodes;
   a well array structure formed from the substrate to define an array of walls defining through-holes which together with the electrodes define a plurality of wells, such that each electrode forms at least in part, a portion of a single well; and
   a plurality of electrical contacts on a backside of the substrate each in contact with a respective electrode for connecting to an electronic circuit.
65. A sensor apparatus as recited in clause 64, comprising an electronic circuit connected to the electrical contacts.
66. A sensor apparatus as recited in clause 64 or 65 wherein the substrate is a thermoplastic substrate.
67. A sensor apparatus comprising:
   a substrate comprising a plurality of vias penetrating through the substrate, at least partially filled with conductive material and forming a plurality of electrodes;
   a well array structure adhered to a frontside of the substrate, said well array structure comprising an array of walls defining through-holes which together with the electrodes define a plurality of wells, such that each electrode forms at least in part, a portion of a single well;
   and an electronic circuit on or connected to a backside of a thermoplastic substrate.
68. A sensor apparatus as recited in clause 67 wherein the substrate has connectors for making electrical contact with an electronic circuit.
69. A sensor apparatus as recited in clause 68 wherein the connectors comprise protrusions from said vias.
70. A sensor apparatus as recited in clause 67 wherein the substrate comprises an electronic circuit formed onto its backside.
71. The sensor apparatus as recited in any of clauses 58 to 70 further comprising an aqueous solution and a plurality of membranes comprising amphiphilic molecules in the wells of the well array structure to encapsulate the aqueous solution therein, thereby forming an array of discrete electrically isolated volumes.
72. The sensor apparatus as recited in clause 71, wherein the aqueous solution is a polar medium.
73. The sensor apparatus as recited in clause 71 or 72, wherein the amphiphilic molecules comprise a hydrophilic head group and a hydrophobic tail group, or a triblock copolymer.
74. The sensor apparatus as recited in any of clauses 58 to 73, wherein the well array structure comprises a base and partitions extending from the base that define the wells and constrain an aqueous solution in a well from contacting aqueous solution in neighbouring wells.
75. The sensor apparatus as recited in clause 74 wherein the partitions comprise inner portions defining inner recesses and outer portions.
76. The sensor apparatus as recited in clause 74 wherein the inner recesses have surfaces having a patterning comprising a plurality of indentations that extend outwardly of the inner recess.
77. The sensor apparatus as recited in clause 75 or 76 wherein the outer portions have surfaces having a patterning comprising a plurality of indentations that extend outwardly of the inner portions.
78. The sensor apparatus as recited in any of clauses 74 to 77 wherein the outer ends of the partition extend in a common plane.

## Claims

1. A method of forming a nanopore sensing device, comprising:
providing a substrate having a surface having an array of electrodes located thereon;
moulding a well array structure having an array of through-holes onto the surface of the substrate such that the array of though-holes and the array of electrodes are aligned; wherein
the substrate and the through-holes of the well array structure define an array of wells, and each electrode is positioned at the base of respective well.

2. The method as claimed in claim 1, wherein the step of moulding comprises:
providing a mould, defining an inverse of the well array structure, on the substrate; and
depositing a mouldant into the mould.

3. The method as claimed in claim 2, wherein the mould comprises an array of protrusions on an underside thereof, and the step of providing the mould comprises:
aligning the underside of the mould with the substrate such that the array of protrusions is aligned with the array of electrodes.

4. The method as claimed in claims 2 or 3, wherein the mouldant is a polymer and the method further comprises:
processing the polymer to cure the polymer and secure the well array structure to the substrate; and optionally wherein
the mouldant is a thermal cure epoxy or UV curable epoxy, and the step of curing optionally comprises: heating the mouldant or exposing the mouldant to UV light.

5. The method as claimed in any of claims 2 to 4, further comprising separating the mould from the mouldant, thereby forming the well array structure.

6. The method as claimed in any preceding claim, wherein the well array structure is moulded directly onto the surface of the substrate; or wherein the array of electrodes is for connecting to or forming part of an electronic circuit.

7. The method as claimed in any of claims 2 to 5, wherein the material of the mould is a thermoplastic, preferably polydimethylsiloxane; and optionally wherein
the mould is an elastomer and the step of providing the mould on the surface of the substrate includes conforming the mould to the surface of the substrate.

8. The method as claimed in any of claims 2 to 5 or 7, wherein the mouldant is a polymer, preferably poly(methyl methacrylate); and optionally wherein
the polymer is dissolved in a solvent before being deposited into the mould; and optionally comprising heating the polymer to evaporate the solvent.

9. The method as claimed in any preceding claim, further comprising providing a patterned adhesive surface on the surface of the substrate, wherein said patterned adhesive surface defines a plurality of portions without adhesive, wherein each electrode is positioned in one of the portions without adhesive; and optionally further comprising:
heating the adhesive surface and/or exposing the adhesive surface to electromagnetic radiation to adhere the well array structure to the substrate; and optionally wherein
providing the patterned adhesive surface comprises exposing a photo-patternable adhesive to light.

10. The method as claimed in any preceding claim, wherein the well array structure has a thickness of between 10 microns and 500 microns, the pitch between the wells is between 10 microns and 1000 microns, and the diameter of the wells is less than 200 microns.

11. The method as claimed in claim 3, wherein the array of protrusions includes a plurality of fins, disposed radially around each protrusion, each fin extending along the length of each protrusion; wherein the step of moulding the wells includes forming a plurality of corresponding fins around a wall of each well.

12. The method as claimed in any preceding claim, further comprising:
populating the wells with a polar medium and forming membranes across the wells; and inserting nanopores in the membranes; or cleaning the electrodes using one of chemical cleaning or plasma treatment.

13. A mould adapted for use in the method of any preceding claims.

14. A master well array structure, for use in forming the mould of claim 13.

15. A nanopore sensor apparatus, comprising:
a substrate;
an array of electrodes located on a surface of the substrate;
a well array structure, moulded from a polymer, comprising a plurality of walls defining through-holes which together with the electrodes define a plurality of wells, such that each electrode forms at least in part, a portion of a single well; and wherein
the array of electrodes form part of the electronic circuit or are connected to an electronic circuit.
